# EUROPEAN PATENT SPECIFICATION

(11) **EP 4 034 055 B1**
(45) Date of publication and mention of the grant of the patent: **19.08.2026**
(21) Application number: 20868854.9
(22) Date of filing: 21.09.2020
(51) Int. Cl.: A61M 13/00, A61F 11/00

(54) **SYSTEMS FOR OPENING EUSTACHIAN TUBES**
SYSTEME ZUM ÖFFNEN VON EUSTACHISCHEN RÖHREN
SYSTÈMES D'OUVERTURE DE TROMPES D'EUSTACHE

(30) Priority: 23.09.2019 US 201962904503 P
(43) Date of publication of application: 03.08.2022
(73) Proprietor: The Board of Trustees of the Leland Stanford Junior University, Stanford, CA 94305-2038 (US)
(72) Inventor: OLDAKOWSKI, Matthew P., Applecross, Australian Capital Territory 6153 (AU); BARTUNEK, Josef, 9052 Gent (BE); BUTLER, Rachel Margaret, Seattle, Washington 98133 (US); DUFFY, Clancy, Como, Australian Capital Territory 6152 (AU); SANTA MARIA, Peter Luke, Emerald Hills, California 94062 (US); SIDELL, Douglas, Half Moon Bay, California 94019 (US); MBAEZUE, Chieloka, Marietta, Georgia 30064 (US); CARRERA, Isabella, Brentwood, California 94513 (US); SILVA ROIG, Frances, La Molina Lima, 15026 (PE); REED, Kelsey, Menlo Park, California 94025 (US)
(74) Representative: Bals & Vogel Patentanwälte PartGmbB
(86) International application number: PCT/US2020/051744
(87) International publication number: WO 2021/061549

(56) References cited:
- US-A- 5 950 631
- US-A1- 2005 000 520
- US-A1- 2012 080 035
- US-A1- 2013 178 716
- US-A1- 2013 211 441
- US-A1- 2019 274 917
- US-B1- 6 565 517

## Description

### CROSS-REFERENCE TO RELATED APPLICATION

Pursuant to 35 U.S.C. §119(e), this application claims priority to the filing date of United States Provisional Patent Application Serial No. 62/904,503, filed September 23, 2019.

### INTRODUCTION

Otitis Media with Effusion (OME) or 'glue-ear' is an extremely common disease in young children (Rosenfeld et al., "Clinical practice guideline: Otitis media with effusion," Otolaryngol Head Neck Surg. (2016)154(Suppl):S1-41). The condition is called an "occupational hazard of early childhood" as it can cause hearing difficulties associated with speech and developmental delay as well as discomfort. The underlying mechanisms involve middle ear inflammation (otitis media) and Eustachian Tube (ET) dilatory dysfunction, which refers to the situation where the ET does not open as it should during swallowing. This condition is associated with poor mid ear ventilation leading to persistent mid ear fluid despite resolution of any underlying acute inflammatory episode. A high prevalence of the condition exists among children ages 1 to 3 years of age.

The current management for this condition includes usually a period of "watchful waiting" involving primary care, or pediatricians prior to being referred for surgery (Rosenfeld, et al., "Clinical practice guideline: Tympanostomy tubes in children," Otolaryngol Head Neck Surg. (2013)149( Suppl):S1-35 and Simon, et al., "Rosenfeld International consensus (ICON) on management of otitis media with effusion in children," Eur Ann Otorhinolaryngol Head Neck Dis. (2018) 135(1S):S33-S39). Surgery involves implanting 'pressure equalization tubes' (PETs) under general anesthesia to ventilate the mid ear through the eardrum.

Surgery is costly (~$3000 per procedure accounting for US$1.8B of spending in the US each year) and limits some activities, such as swimming, and is consequently very unattractive to parents. PETs often fail, up to 20%, requiring a new surgery. They are also associated with complications which potentially lead to further hearing loss than the conditions they were attempting to prevent. These complications include recurrent discharge due to bacterial biofilms forming on the surface (4% incidence), blocked tubes needing replacement (7%), induction of tympanic granulation tissue (2% incidence) and cholesteatoma formation (7% incidence) (Barakate et al., "Bacterial biofilm adherence to middle-ear ventilation tubes: scanning electron micrograph images and literature review," J Laryngol Otol. (2007)121(10):993-7; Groblewski & Harley, "Medial migration of tympanostomy tubes: an overlooked complication," Int J Pediatr Otorhinolaryngol. (2006) 70(10):1707-14; and Kay et al., "Meta-analysis of tympanostomy tube sequelae," Otolaryngol Head Neck Surg. (2001)124(4):374-80).

An alternative to these silastic tubes has the potential to reduce approximately $760 MIL in costs to the health care system annually. This market assessment is based on the incidence of complications associated multiplied by the costs to the health care system for management of each complication which ranges from $2700-$12600 per complication (Cullen et al., "Ambulatory surgery in the United States 2006," Natl Health Stat Report. (2009) 28(11):1-25; Gates, "Cost-effectiveness considerations in otitis media treatment," Otolaryngol Head Neck Surg. (1996)114(4):525-30; O'Brien, et al., "New vaccines against otitis media: projected benefits and cost-effectiveness," Pediatrics. (2009)123(6):1452-63; Schappert & Rechtsteiner, "Ambulatory medical care utilization estimates for 2006," Natl Health Stat Report. (2008)(8):1-29; Coyte et al., "The economic cost of otitis media in Canada," Int J Pediatr Otorhinolaryngol. (1999) 49(1):27-36; Todd. "Audit of the incidence of persistent perforation of the tympanic membrane following T-tube removal or extrusion," J Laryngol Otol. (1993)107(7):590-2; and Todd, "Audit of the incidence of persistent perforation of the tympanic membrane following grommet removal or extrusion," J Laryngol Otol. (1993)107(7):593-6).

Surgical intervention guidelines emphasize shared decision making with parents of children with OME. Recommendations endorse a period of watchful waiting in general children population with repeat 3-monthly evaluation (it is estimated that there are nearly 670,000 tympanostomy tube insertions a year in children in the US (Rosenfeld, et al., "Clinical practice guideline: Tympanostomy tubes in children," Otolaryngol Head Neck Surg. (2013) 149(Suppl):S1-35)). This period provides an ideal window for a disruptive approach. During 'watchful waiting' there is no effective medical treatment available. During the surveillance period, parents and clinicians may use autoinflation of the Eustachian tube by nasal pressurization to ventilate the mid ear (EarPopper/Eustachii and Otovent) (Perera et al., "Autoinflation for hearing loss associated with otitis media with effusion (Review)," Cochrane Database of Systematic Reviews (2013); "Otovent nasal balloon for otitis media with effusion," Medtech Innovation Briefing, March 2016, available at: https-//www.nice.org.uk/advice/mib59). This approach is safe, but these products are not widely adopted due to children compliance and usability issues, difficulty in confirming whether the treatment has been successfully performed and as a result poor clinical evidence of efficacy (potentially due to the first two issues). Furthermore, the inconveniences of the use of these devices limit their acceptability to children and families. The EarPopper/Eustachii require the parent to hold the device to the child's nostril, hold the other nostril closed and press a button to initiate air flow timed with a swallow of some liquid. The Otovent requires the child to blow up a balloon with their nose to create a partial Valsalva. It is onerous for parents to do this therapy multiple times a day for 7 weeks or longer in the target age group (i.e., 1 to 5 year old children). Furthermore, neither the EarPopper nor Otovent product is specifically recommended for children in the high prevalence group of children ages 1 to 3 years old (Otovent is available only for children ages 3 years and up (Otovent Product Information FAQ, available at: https://www.otovent.co.uk/buy-otovent/otovent/); the EarPopper has been examined in children within the age range of 4 to 11 (Arick, D.S. and S. Shlomo, Nonsurgical Home Treatment of Middle-Ear Effusion and Associated Hearing Loss in Children, Part I: Clinical Trial," ENT Journal, Vol. 84, No. 9, Sept. 2005)).

Reference US2005000520A1 discloses an apparatus and method for reducing middle ear fluid and equalizing middle ear pressure in infants and toddlers via the coordinated actions of swallowing and forcing air into the nostril of the child.

Reference US2013178716A1 discloses an apparatus for automatically applying a pneumatic overpressure phase in the Eustachian tube and/or in the nasal fossae of a patient through at least one nostril at the moment of swallowing, the apparatus consisting of a gas generator, a gas reservoir, a detection-fluid and control circuit, a sealed plug in the nostrils equipped with a control nozzle, a device for measuring at least one characteristic value of a flow of gas during swallowing, a device for automatically comparing the characteristic value with at least one pre-set value and a device for informing the patient whether the swallowing action is being correctly performed.

Reference US2013211441A1 discloses a device for obtaining an equalization of a negative pressure in the middle ear, the device comprising a facemask, an air pump and an air reservoir, all connected to a connecting tube.

In addition to children with OME, other conditions also benefit from mechanisms for opening the eustachian tubes of a subject, such as, for example, subjects with eustachian tube dysfunction, OME in adult subjects, subjects with recurrent acute otitis media, subjects who are divers with ear discomfort, subjects who are flight attendants or other airline personnel with ear discomfort or children seeking preventive measures due to being at high risk for OME.

### SUMMARY OF THE INVENTION

The present invention provides a system for opening an eustachian tube of a subject as defined in claim 1. Further preferred embodiments of the present invention are defined in the dependent claims.

### SUMMARY OF THE DISCLOSURE

Thus, there is a need for an improved and useful system and method for opening the eustachian tubes, particularly in children with, or at risk of, Otitis Media with Effusion. This disclosure provides such a new and useful system.

Systems and methods for opening the eustachian tubes of a subject are provided. Aspects of the system according to the invention include: a swallow inducer; a nasal passage seal; a pressure source configured to apply positive pressure to a sealed nasal passage; a sensor configured to detect a parameter indicative of a swallow resultant palate closure; and a controller operably coupled to the pressure source and the sensor, wherein the controller is configured to cause the pressure source to apply positive pressure to a sealed nasal passage upon detection by the sensor of the parameter indicative of a swallow resultant palate closure. Also provided but not part of the invention are methods of using the systems to open eustachian tubes. The systems and methods find use in a variety of different applications, e.g., the treatment of a subject for Otitis Media with Effusion (OME), subjects with eustachian tube dysfunction, subjects with recurrent acute otitis media, subjects who are divers with ear discomfort, subjects who are flight attendants or other airline personnel with ear discomfort or children seeking preventive measures due to being at high risk for OME.

### BRIEF DESCRIPTION OF THE FIGURES

The disclosure may be best understood from the following detailed description when read in conjunction with the accompanying drawings. Included in the drawings are the following figures:
FIGS. 1A to 1D provide various views of a handheld device in accordance with an embodiment of the disclosure.
FIG. 2 provides various views of a handheld device in accordance with another embodiment of the disclosure.
FIG. 3 provides various views of different handheld devices in accordance with various embodiments of the disclosure.
FIG. 4 provides various views of a handheld device in accordance with another embodiment of the disclosure.
FIGS. 5A to 5B provide various views of a handheld device in accordance with another embodiment of the disclosure.
FIGS. 6A to 6K provide various views of a handheld device in accordance with another embodiment of the disclosure.
FIGS. 7A and 7B provide various views of mask device that does not include an integrated swallow inducer, in accordance with another embodiment of the disclosure.
FIGS. 8A to 8D provide various views of an under the nose mask for use in embodiments of the disclosure.
FIG. 9 depicts a control diagram for the operation of an embodiment of the system in which the system transitions between a "sleep mode," a "sensing mode," a "therapy mode," a "success mode," a "training mode" and a "trouble shooting mode."
FIG. 10 depicts a control diagram for the operation of a "training mode" for an embodiment of the system.
FIG. 11 provides views of a remote device with various GUIs, in accordance with an embodiment of the disclosure.
FIG. 12 provides views of a remote device with various game type GUIs, in accordance with an embodiment of the disclosure.
FIGS. 13A and 13B illustrate operation of a device during use, in accordance with an embodiment of the disclosure.

### DETAILED DESCRIPTION

Systems and methods for opening the eustachian tubes of a subject are provided. Aspects of the system according to the invention include: a swallow inducer; a nasal passage seal; a pressure source configured to apply positive pressure to a sealed nasal passage; a sensor configured to detect a parameter indicative of a swallow resultant palate closure; and a controller operably coupled to the pressure source and the sensor, wherein the controller is configured to cause the pressure source to apply positive pressure to a sealed nasal passage upon detection by the sensor of the parameter indicative of a swallow resultant palate closure. Also provided but not part of the invention are methods of using the systems to open eustachian tubes. The systems and methods find use in a variety of different applications, e.g., the treatment of a subject for Otitis Media with Effusion (OME), subjects with eustachian tube dysfunction, subjects with recurrent acute otitis media, subjects who are divers with ear discomfort, subjects who are flight attendants or other airline personnel with ear discomfort or children seeking preventive measures due to being at high risk for OME.

Before the present disclosure is described in greater detail, it is to be understood that this disclosure is not limited to particular embodiments described, as such may, of course, vary. It is also to be understood that the terminology used herein is for the purpose of describing particular embodiments only, and is not intended to be limiting. The scope of the present invention is limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, representative illustrative methods and materials are now described.

The citation of any publication is for its disclosure prior to the filing date and should not be construed as an admission that the present disclosure is not entitled to antedate such publication by virtue of prior invention. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope of the present disclosure. Any recited method can be carried out in the order of events recited or in any other order which is logically possible.

### OVERVIEW

As summarized above, the present disclosure provides systems and methods for opening eustachian tubes of a subject. As such, systems and methods are provided for opening the canals on each side of the face that run from the back of the nose and upper throat to the middle ear. By "opening" is meant that the systems and methods may be used to transition eustachian tubes from a closed state where gas and liquid cannot transit the tubes between the middle ear to the back of the nose and upper throat to an open state where gas and liquid can transit the tubes between the middle ear to the back of the nose and upper throat. As such, the systems and methods may result in insufflating the eustachian tubes of the subject. The subject is generally a human subject and may be male or female. While the subject may be of any age, in some instances the subject is not an adult, such as a baby, toddler, juvenile, child, etc.

### SYSTEMS

Aspects of the systems include: a swallow inducer; a nasal passage seal; a pressure source configured to apply positive pressure to a sealed nasal passage; a sensor configured to detect a parameter indicative of a swallow resultant palate closure; and a controller operably coupled to the pressure source and the sensor, wherein the controller is configured to cause the pressure source to apply positive pressure to a sealed nasal passage upon detection by the sensor of the parameter indicative of a swallow resultant palate closure. Additionally, in certain embodiments, the system may also include a sensor operably coupled to the controller and configured to detect whether the system is currently in use. Each of these components is now described further in greater detail.

### Swallow Inducer

A component of the systems is a swallow inducer, which is configured to cause a subject to swallow. The swallow inducer may be an ingestible composition associated with an oral delivery device for delivery of the ingestible composition to the mouth of the subject, where upon the subject swallows the ingestible composition. The nature of the ingestible composition may vary, where ingestible compositions include, but are not limited to, liquids, e.g., water, juices, nutritional drinks, energy drinks, sodas, etc.; and solids, e.g., foods, candies, etc. The oral delivery device may vary depending on the nature of the ingestible composition. Where the ingestible composition is a liquid, the oral delivery device includes a container for the liquid, e.g., bottle, cup, etc., in operable, e.g., fluidic communication with, an outlet of an oral mating device, e.g., a mouthpiece, such as a mouthpiece configured to be sucked on by a subject, e.g., a nipple, sip cup lid, straw, etc. The container may be configured to hold any desired volume of liquid ingestible composition, where in some instances the container is configured to hold a volume ranging from 5 mL to 2 L, such as 150ml to 500 ml. Where the ingestible composition is a solid, e.g., a hard candy composition, a popsicle, etc., the oral delivery device may be a handle or analogous structure for holding the ingestible composition to the mouth of the subject.

### Nasal Passage Seal

Systems of the disclosure also include a nasal passage seal, which is configured to seal the nasal passage of the subject with an airtight seal. The nasal passage seal may vary as desired. In some instances, the nasal passage seal is an extra-nostril seal, by which is meant that it provides for sealing of the nasal passage using a structure that is not present in a nostril during use. Examples of extra-nostril nasal passage seals include, but are not limited to, nasal masks or analogous structures that are configured to cover the outside of the nose or a portion thereof in a manner that provides for an airtight sealed nasal passage. Where the nasal passage seal is a nasal mask, the dimensions of the nasal mask may vary, where in some instances the nasal mask has a shape and dimensions to cover a child's nose. In some instances, the nasal mask has a roughly isosceles triangular shape configured to fit over a nose, e.g., with a height ranging from 20 mm to 80 mm, such as 30 mm to 50 mm, and a base ranging from 20 mm to 90 mm, such as 30 mm to 60 mm. The depth of the mask may also vary as desired, ranging in some instances from 2 mm to 60 mm, such as 20 mm to 60mm.

Still other examples of extra-nostril nasal passage seals include, but are not limited to, under the nose masks. An under the nose mask may be configured to engage and seal, or cap, the outside of each nostril. Where the nasal passage seal is an under the nose mask, the dimensions of the under the nose mask may vary, where in some instances the under the nose mask has a Y-shape, where the upper part of the "Y" is a semicircular shape so as to wrap around the underside of the subject's nose, thereby interfacing with and sealing each nostril. In some cases, the size of the semicircular shaped under the nose mask is configured to fit under a nose, e.g., with a diameter ranging from 1 mm to 20 mm, such as 3 mm to 15 mm or 5 to 10 mm or a width ranging from 1 mm to 20 mm, such as 3 mm to 15 mm or 5 to 10 mm and a height ranging from 1 mm to 10 mm, such as 1 mm to 5 mm or 1 mm to 3 mm (where of the semicircular shape corresponds to the thickness of the mask at this point, i.e., the length of the extruded hole). The depth of the under the nose mask may also vary as desired, ranging in some instances from 5 mm to 20 mm, such as 5 mm to 10 mm. When the under the nose mask is semicircular in shape, in some cases, the ends of the semicircular shape are flaps that help align the nose mask and the subject's nose. That is, when applying the under the nose mask to the nose, the semicircular shape of the nose mask, including the two ends of the semicircular shape - or the two flaps - may urge the nose into the center of the nose mask such that the nose mask can better engage with, and seal, each nostril. In some cases, under the nose masks may comprise one or more holes allowing fluidic communication between the sealed nasal passage and a pressure source of the system. In some cases, under the nose masks may comprise exactly one hole for interfacing between the system, such as the pressure source of the system, with both nostrils. That is, a single hole in the under the nose mask may be used for both of the subject's nostrils.

In some cases, nose masks, such as under the nose masks, may comprise dual durameter nose masks. By dual durameter nose mask, it is meant a nose mask made from materials with different degrees of hardness. In some cases, softer materials (i.e., materials for which a lesser amount of force is required to deform or otherwise change the shape of the material) are used on the inner side (i.e., the side that is proximal to the subject) of a dual durameter nose mask, and harder materials (i.e., materials for which a greater amount of force is required to deform or otherwise change the shape of the material) are used on the outside side (i.e., the side that is distal to the subject) of the mask. By inner side of the mask, it is meant the side of the mask that engages with the nostrils, in other words, the side of the mask that is proximal to the subject's nose. By softer materials, it is meant any convenient material that exhibits desirable deformation properties, such that the inner side of the nose mask deforms to a desirable degree in order to substantially conform to the nose and nostril area, thereby sealing the nostrils, when the nose mask is held against the subject's nose during normal use of the system. Materials of interest may include, but are not limited to, silicone, such as EcoFlex silicone or molded EcoFlex silicone gel or similar, or, in other cases, elastic resins. Materials of interest may be food safe materials and/or may be temperature resistant, such as, for example, dishwasher safe. By outer side of the mask, it is meant the side of the mask that does not engage with the nostrils but instead may connect the dual durameter nose mask with the rest of the system, in other words, the side of the mask that is distal to the nose. In some embodiments, when applied to the nose, the combination of the soft inner material with the hard outer material of dual durameter under the nose mask causes the inner material to deform by spreading out or flattening, thereby conforming to the shape of each of the subject's nostrils under the subject's nose, and form a seal with each nostril by completely covering the outside of each nostril. That is, the inner material may be deformed into a shape that conforms to the subject's nostrils and nose when the under the nose mask is engaged under the subject's nose such that the inner material is compressed between the harder outer material and the nose. Use of an adequately deformable softer material for the inner or proximal side of the dual durameter nose mask may facilitate greater effectiveness of the system across a broader population of users with a wider variation of shapes and sizes of noses and nostrils.

In some embodiments that comprise under the nose masks, under the nose masks are attached other components of the system by a flexible joint. In embodiments where under the nose mask is Y-shaped, the bottom portion of the "Y" may attach to the rest of the system via a flexible joint. For example, in certain embodiments, an under the nose mask may be attached to the top or the lid portion of a cup used to deliver liquid to the user to induce swallowing. By flexible joint, it is meant that the under the nose mask is configured to rotate as needed such that its position is adjusted when the system is in use by the subject to better conform to the nose and, in particular to the nostrils, so as to better seal the nostrils. In some cases, the flexible joint may be configured to rotate in any direction of the plane normal to the flexible joint used to attach the under the nose mask to the system, such as in a substantially downwards, substantially rightwards, substantially leftwards or substantially upwards direction, when viewing the under the nose mask along the axis through the flexible joint. In other cases, the flexible joint may be configured to rotate in a range of directions of the plane normal to the flexible joint used to attach the under the nose mask to the system, such as a range of 5° or more, 45° or more, 90° or more, 180° or more, 270° or more, when viewing the under the nose mask along the axis of the flexible joint. In some cases, the flexible joint can rotate up to 90° or more relative to the axis through the flexible joint, such as 5° or more, 15° or more, 30° or more, 45° or more, 60° or more or 90° or more. When a flexible joint is used in conjunction with a dual durameter under the nose mask, the flexible joint may be comprised of the same material that comprises the harder outside of the mask, or in other cases, may be comprised of yet another material. In some cases, the flexible joint may be a flexible element. The flexible joint or flexible element may be configured to allow rotation of the under the nose mask to account for variation in facial angulation. The flexible joint may address a need that a Y-shaped under the nose mask may need to be positioned at a different angle relative to the rest of the system for different nose sizes and geometries. In some cases, the flexible nose mask may be mounted on the rest of the system at an angle that is substantially orthogonal to the axis of the joint attaching the under the nose mask to the system. In other cases, the flexible nose mask may be mounted on the rest of the system at a non-orthogonal angle, such as 1° or more, 5° or more, 10° or more or 30° or more.

Instead of an extra-nostril nasal passage seal, such as a nasal mask or an under the nose mask, e.g., as described above, systems of the disclosure may include intra-nostril nasal passage seals, where such sealing elements are introduced into the nostril in order to provide for the desired airtight nasal passage seal. Intra-nostril seals may have any convenient configuration that provides for insertion of at least a portion of the component in to the nostril to provide for the desired seal. In some instances, the portion of the intra-nostril seal that is configured to be inserted into the nostril may have a roughly cylindrical shape, e.g., with a diameter ranging from 2.0 mm to 15 mm, such as 4 mm to 6 mm.

The nasal passage seal, which may be referred to as a nose-piece, functions to engage and seal the nasal cavity to allow nasal cavity pressurization to occur. The nose-piece can engage any location on the face including inside the nostrils (like a nasal probe), the bottom of the nostrils (like the EarPopper or a CPAP nasal pillow), around the nose (like a nasal CPAP mask), around the entire face like a full-face anesthesia mask or around the entire head (like a helmet). In order to facilitate an effective seal around the nose the nose-piece may be moldable to the subject, 3D printed to suit the subject's anatomy, provided with multiple different sizes, or adjustable. Methods of increasing the sealing pressure include multiple seals, suction cup, a partially inflated bag construction, a pressure-based adhesive, static electricity-based sealing and gels to improve sealing.

### Pressure Source

Systems of the disclosure further include a pressure source configured to apply positive pressure to a sealed nasal passage. The pressure source is configured to provide for a desired elevated pressure in the sealed nasal passage during use, e.g., a pressure sufficient to result in opening of the eustachian tubes. While the desired elevated pressure in the sealed nasal passage that the pressure source is configured to provide may vary, in some instances the desired elevated pressure ranges from 0.3 PSI to 10 PSI, such as 0.5 PSI to 1 PSI, or 3 PSI to 4 PSI, or 5 PSI to 6 PSI. The pressure source may be configured to provide for the desired elevated pressure for a set duration, where in some instances the set duration ranges from 0.05 s to 2 s, such as 0.2 s to 0.5 s or 0.4 s to 0.5 s or 0.9 s to 1 s. Pressure sources of the systems may vary, where examples of pressure sources include, but are not limited to: air pumps, pressurized cannisters, or balloons, etc. Where the pressure source includes a pressurized or compressed gas, e.g., as present in a pressurized cannister, the pressurized gas may vary as desired, ranging from air, helium, argon, water vapor, and the like. Where desired, the system may include a component, e.g., heater and/or cooler, to modulate the temperature of the gas, e.g., to a temperature ranging from -10 to 40 °C. In some instances, the pressurized gas may include an active agent, such as an active pharmaceutical ingredient, e.g., where the system is employed to deliver a drug to the eustachian tubes. In some instances, the system may include a component configured to provide a pleasurable smell in the gas that is pressurizing the nasal cavity, such as aromatic oils. The pressure source may be operably coupled to the nasal passage seal to provide for the desired increase in nasal passage pressure during operation. For example, where the pressure source includes a source of pressurized gas, e.g., a pressurized cannister, the pressure source may be coupled to a nasal passage site of the nasal passage seal by a gaseous passageway, e.g., tube or lumen, which provides for passage of the pressurized gas from the source into the nasal passage during use. A reversable closure, e.g., valve, may be present to open and close the passageway to provide for delivery of pressurized gas to the sealed nasal passage when desired, e.g., during a predetermined time, such as described below.

In some instances, the pressure source may be configured to provide a specified amount of constant pressure (i.e., a baseline "low flow mode") upon an embodiment of the system detecting that a specified event has occurred. For example, the pressure source may provide "low flow mode" pressure after an embodiment of the system detects that it has been picked up. The pressure source may be configured to provide a further elevated pressure (i.e., a "high flow mode"), such as that described above, e.g., a pressure sufficient to result in opening of the eustachian tubes, when already applying pressure in "low flow mode."

### Sensor

Systems of the disclosure may also include a sensor configured to detect a parameter indicative of a swallow resultant palate closure. By swallow resultant palate closure is meant the closure of the palate as a result of swallowing by the subject. By parameter indicative of a swallow resultant palate closure is meant a detectable change, such as in change in pressure, structure, sound, etc., that may be employed to infer the closure of the palate as a result of swallowing. The sensor may vary as desired, where suitable sensors include, but are not limited to, pressure sensors, e.g., where the parameter is a rise in pressure in the nasal passage above a predetermined threshold, e.g., a predetermined threshold ranging from, e.g., 0.05 PSI to 0.7 PSI, a sound sensor, e.g., to detect sound produced by swallowing, an optical sensor, e.g., to detect an anatomical change indicated of swallowing, e.g., laryngeal elevation resultant from swallowing, tongue movement during swallowing, etc.

### Controller

Systems of the disclosure further include a controller configured to cause the pressure source to apply positive pressure to a sealed nasal passage of the subject for a predetermined application period upon detection by the sensor of the parameter indicative of a swallow resultant palate closure. As such, the controller is operatively coupled to the sensor, and upon receipt of the parameter indicative of a swallow, e.g., as described above, causes the operatively coupled pressure source to apply positive pressure to the sealed nasal passage so as to open the eustachian tubes. The predetermined application period may vary, ranging in some instances from 0.05 s to 2 s such as 0.2 s to 0.4 s or 0.4 s to 0.5s or 0.9 s to 1 s. The controller may be made up of one or more functional blocks which act in concert to perform the desired function, e.g., operation of the system. A given controller may be implemented as hardware, software or a combination thereof. In some instances, controller may include a circuitry element, such as an integrated circuit. When present, integrated circuits may include a number of distinct functional blocks, where the functional blocks are all present in a single integrated circuit. By single integrated circuit is meant a single circuit structure that includes all of the different functional blocks. As such, the integrated circuit is a monolithic integrated circuit (also known as IC, microcircuit, microchip, silicon chip, computer chip or chip) that is a miniaturized electronic circuit (which may include semiconductor devices, as well as passive components) that has been manufactured in the surface of a thin substrate of semiconductor material.

In some instances, the controller may be further configured to provide indicative feedback that conveys information about the controller and/or an embodiment of the system. The indicative feedback mechanism may be made of one or more discreet components, as desired. While the indicative feedback may vary, in some instances the feedback includes lights, sounds, or vibrations, e.g., an LED light, where the feedback is indicative of states of the controller such as sleep, standby, operational states, communication, successful treatment, or system errors.

In some instances, the controller may be configured to provide on-device storage. The controller may be configured to write various treatment-related data to the on-device storage, such as, for example, the time of the last treatment. The controller may also be configured to record sensor data associated with each attempted treatment. Data stored on the device may use a microprocessor RAM or an SD card or other technologies. The data may be raw pressure data or compressed summary data.

### Communications Module

Systems of the disclosure may further include a communications module, which module is operably coupled to one or more components of the system and provides for data transfer therefrom to another component, e.g., an external device, such as a computer, tablet, smart phone, etc. The communications module may be configured to provide for the transfer of data in a wired or wireless mode, as desired. For example, the communications module may be configured to transfer data, e.g., with a networked device, while being used. The communication may be wireless or wired, as desired. Communications modules of the systems may be configured, e.g., via hardware and/or software implementation, to perform desired communications functions, e.g., to receive data from a system element, to transfer data, e.g., to a USB port for wired communications or a wireless transmitter for wireless communications, etc. Communications modules (as well as any other modules described herein, such as actuator controller modules, etc.) are made up of one or more functional blocks which act in concert to perform a particular function, which is the purpose of the module. A given communications module may be implemented as hardware, software or a combination thereof. In some instances, the communications module may include a circuitry element, such as an integrated circuit, e.g., as described above.

In certain instances, the controller includes an updatable control module, by which is meant that the controller is configured so that one or more control algorithms of the controller may be updated. Updating may be achieved using any convenient protocol, such as transmitting updated algorithm data to the controller using a wire connection (e.g., via a USB port on the device) or a wireless communication protocol. The content of the update may vary. The update information may also include general functional updates, such that the controller can be updated at any desired time to include one or more additional software features and/or modify one or more existing programs of the device. The update information can be provided from any source, e.g., a separate update device, the internet, etc.

### Power Source

Where desired, systems of the disclosure may include a variety of different types of power sources that provide operating power to the system components, e.g., as described above, in some manner. The nature of the power source may vary, and may or may not include power management circuitry. In some instances, the power source may include a battery. When present, the battery may be a onetime use battery or a rechargeable battery. For rechargeable batteries, the battery may be recharged using any convenient protocol, including, but not limited to, wireless charging protocols such as inductive charging. In some applications, the actuator may have a battery life ranging from 15 seconds to 7 weeks, such as 15 minutes to 1 hour or 1 day to 1 week.

### Remote Device

As reviewed above, in some instances the systems include a remote device that is in communication with the controller, e.g., via a wired or wireless communication. By "remote" is meant a location apart from the components that are interacting with the subject during use. For example, a remote location could be another location, e.g., different part of a room, different room, etc., in the same vicinity of the subject, another location in a vicinity different from the subject, e.g., separate portion of the same building, etc., another location in a different city, state, another location in a different country, etc. As such, when one item is indicated as being "remote" from another, what is meant is that the two items are at least in different locations, not together, e.g., are 1 to 5 or more feet apart, such as 10 or more feet apart, including 25 or more feet apart. While the remote device may vary, in some instances the remote device includes a computer, a processor and a display component, e.g., digital readout, screen, monitor, etc., where the processor is configured to display data received from the communications module on the display device, in a graphical user interface (GUI) etc. While the nature of the remote device may vary, e.g., as described above, in some instances the remote device is a desktop computer, laptop computer, tablet or smart phone.

Where the system includes a remote device, the remote device may be configured to display data, e.g., via a GUI, to one or more stakeholders, such as the subject, parents or other caregivers of the subject, health care practitioners, etc. In some instances, the remote device is configured to display therapeutic data to a stakeholder, such as data regarding administration of positive pressure to a sealed nasal passage, etc. Therapeutic data displayed on the remote device may also include, for example, the total number of therapies (i.e., the system registers that positive pressure was applied to a sealed nasal passage) applied during a specified time period, such as within a one hour time period or since the remote device was last engaged with by a user, the number of therapies per session, the number of incomplete therapies (i.e., incomplete pressure events meaning, for example, the pressure of the nasal passage failed to reach a threshold pressure, potentially indicating poor sealing of the nasal passage) or the ratio of incomplete therapies to complete therapies (i.e., complete pressure events meaning, for example, the pressure of the nasal passage successfully reached a threshold pressure), or the number of upside down drinking events (potentially preventing therapy).

In some instances, the remote device is configured to provide one or more engagement elements, meaning aspects to make the use of an embodiment of the system more engaging for the subject and/or the caregiver. An example engagement element would be to display one or more gamification aspects to a stakeholder, e.g., the subject, to keep the subject engaged in the therapy, or to educate the subject and/or the caregiver about the therapy or to educate and/or resolve issues related to the correct and/or effective use of the system. The gamification aspects of the display may be implemented and presented to the stakeholder through any convenient protocol, for example a software application executed on the remote device (an "app").

The gamification aspects of the app may include techniques and functionality intended to increase user engagement, educate the subject and the caregiver and to create a sense of purpose and ownership of the therapy. Gamification is targeted at both children, who are recipients of the therapy, and caregivers.

The gamification aspects of the app may be incorporated during specific modes of operation of the system and may include a 'training mode' (where an on-screen character leads the user through instructions for using the device for the first time) or a 'trouble-shooting mode' (where an on-screen character provides instruction and encouragement to solve a problem). Other examples of gamification elements are described below.

Gamification aspects of the app targeted at a child receiving therapy may be provided and, as described above, may be designed to increase the engagement of the child by creating an enjoyable experience and a sense of ownership. Such aspects may include an on-screen character that the child can choose from several options. The on-screen character would provide:
- Silly antics such as protesting when the child shakes the device;
- Encouragement, praise and instruction (including during 'training mode' of the app);
- Consistent animation when the therapy is completed (such as the on-screen character sneezing);
- Change in character status (such as size, appearance, complexity, customizability) when the child reaches certain therapy-related milestones. Such changes in character status may include:
   earning badges for certain therapy-related milestones such as completing therapy of a certain duration, etc.; digital rewards for completing each session's therapy such as stamps, stickers, and costume elements that can be applied to the on-screen character;
   special treatment such as the on-screen character changing color, changing clothes, becoming more ornamented, etc. for bonus therapy (therapy completed in addition to that corresponding to a daily goal).

Gamification aspects of the app targeted at a caregiver may be provided as well to increase the engagement of the caregiver by creating a sense of achievement and informing the caregiver, in particular, providing information to help create a sense that the treatment program is feasible. Such aspects may include: an option to personalize an in-app profile; progress graphics including the session's and the day's progress goals and a calendar showing how much progress was achieved within a certain time period, for example a month of progress at a time (goals can be customizable by the caregiver within certain set limits); notifications in the form of animations and/or vibrations and/or other notification techniques when progress goals are close to being met or have been met or have been exceeded, or alternatively, when progress has fallen behind that required for a specified goal or when the device has detected the subject may not be using the system effectively (e.g., a high ratio, or an increasing ratio, of incomplete therapies to complete therapies); encouragement in the form of in-app messages or animations when a 'streak' of therapy is being formed and/or maintained (i.e., when the subject adheres to a consistent pattern of treatments, such as using the system at least once a day, where such pattern is custom izable and can be specified by the caregiver); bonus therapy (therapy completed in addition to a daily goal) may receive a notification message or animation and may be denoted in a special way (such as a star) on the progress calendar.

Further engagement elements may consist of awarding points to the subject for use of the device. Awarded points may be used to "score" the subject's use of the device such that higher scores are acknowledged and potentially shared within a community, including an online community, of subjects using embodiments of the system. Alternatively, awarded points may be given a monetary value such that points can be exchanged for prizes in the form of retail purchases.

### Aligner

In some instances, the systems include an aligner, which is configured to provide for proper spatial orientation of the various components, e.g., nasal passage seal, mouthpiece, etc., of the system with the subject during use. The aligner may be made of one or more discreet components, as desired. Various different types of alignment components may be present in the systems, where examples of such alignment components include, but are not limited to: a bite piece that can only be used in one orientation, a chin blocker to stop the subject from pivoting their chin backwards, a small bite nozzle (e.g., having a length from 0 mm to 15 mm) to bring the face of the subject close to the device, nose blocking elements, an accelerometer-based system to prevent the device from operating if drinking with the head back, or with the bottle upside down and the chin inside the mask or otherwise using inappropriately, etc.

### Additional Sensors

In some instances, the system may include one or more additional sensors. For example, the system may include an additional sensor configured to detect whether the system is being used and/or to detect the orientation of the system. Such a sensor may be comprised of an accelerometer configured to, among other things, signal accordingly when the system is not moving and therefore not in use. An embodiment that includes such a sensor may offer advantages such as facilitating a "ready to go" or "sleep" mode with reduced energy usage, thereby extending the battery life, or reducing the need for caregiver interventions to turn the device on or off for a child subject.

Where desired, the system may include a spatial positioning sensor. Such a spatial position sensor may be configured to provide information about the system and in particular may be configured to provide information about the controller. Such a spatial positioning sensor may vary in nature, in some instances it may be capable of measuring acceleration, orientation and/or jerk, e.g. an accelerometer, gyroscope or tilt switch, where the sensed information is indicative of the state of the system and, for example, in particular the controller, such as "still," "in motion," "inverted," "freefall" or "impacted."

### Additional Aspects

Where the system is employed for active agent delivery, instead of including the active agent in a pressurized gas, e.g., as described above, the system may include a separate active agent composition source that is operably coupled so as to be delivered to the sealed nasal passage during use. The active agent composition source in such instances may be a separate container which is connected, e.g., via tube or analogous structure, to the pressure source, the nasal passage seal, etc., so as to be delivered to the sealed nasal passage during use. The active agent composition may be a solid, e.g., particles, or liquid, as desired, and may include a variety of different active agents in any convenient delivery vehicle. Examples of active agents that may be present include, but are not limited to: anti-bacterial and anti-viral agents, anti-inflammatory agents, corticosteroids, decongestants, aerosols, biologics, and the like.

Systems of the disclosure may vary. In some instances, the systems are handheld devices that include all of the disparate components of the device integrated into a single handheld unit. In such embodiments, the handheld devices are configured to be held easily in the hand of a human, such as a non-adult human, e.g., as described above. Accordingly, the devices may have a configuration that is amenable to be held by a non-adult human, e.g., with both hands or a single hand. The weight of the devices may vary, and in some instances may range from 50 to 2000 g, such as 300 to 500 g. Handheld devices of the disclosure may have any convenient configuration, where examples of suitable handle configurations are further provided below.

Systems of the disclosure may also be made up of two or more disparate units that work together to provide the desired functionality. For example, the system may include a first unit that includes the nasal passage seal, the sensor and the controller, and a second unit that includes the swallow inducer, e.g., a popsicle, hard candy, etc., such as exemplified below.

In other embodiments, the system may include a first unit that includes electrical components (e.g., a pressure source, a sensor, a controller, a communications module and/or a power source) and a second unit that includes all other components (e.g., a bottle, a straw, a nasal passage seal, facial alignment features, a nozzle, a nasal mask, handles, etc.) of the system. In such embodiments, when components of the second unit of the system are assembled, such assembled second unit may itself form a functioning sippy cup. That is, the components of the second unit may be assembled and utilized as a sippy cup (such as a cup designed to be held by an infant, toddler or young child to deliver liquid) completely independent of the first unit. By completely independent of the first unit, it is meant that the second unit may be assembled without the first unit to form a functioning sippy cup.

In embodiments of the system that comprise a cup, such as, for example, the second unit of the system as described above, the system may further include a suction cup configured to removably attach the system to a tabletop. For example, a system comprising a cup element may be configured such that a suction cup is affixed to the bottom of the cup element, which may be used to hold the cup onto a table.

In some cases when the system comprises a disparate unit that comprises electronics, such as a disparate unit that comprises electrical components, the system is configured so that the electronics are inactive until the system is assembled. That is, the system may be configured such that the electronics remain off or in an inactive state until the system, including the disparate unit that comprises electronics, is assembled. In other words, the system may be configured such that assembly of the system, including assembly of a disparate unit comprising electronics into the system, may be tantamount to an "on" switch or an "activate" switch for the electronics or electrical components, such that the system is turned on by assembly. In such embodiments, the system may be configured such that the final step in assembling the system is combining a subassembly of electronic components with a subassembly of all other elements of the system, such that turning on the system by adding the electronics only occurs when the system is fully assembled.

Where desired, systems of the disclosure may further include an aural element, e.g., configured to be placed over the ears to create pressure (either static or alternating with or without a vacuum) on the eardrum during eustachian tube opening to dislodge the effusion, a warming element to warm the effusion through the eardrum in order to decrease the viscosity of the effusion, etc.

Where desired, systems of the disclosure may include one or more design elements configured to engage a subject and/or make use of the system by the subject more enjoyable. Examples of such design elements include, but are not limited to: a pleasurable smell, a pleasurable color or decorative design, a pleasant tasting ingestible liquid or solid comprising the swallow inducer, entertaining gamification aspects in conjunction with the remote device, rewards-based incentives based on correct or effective use of the device, a nice vibration, a pleasant music or other entertaining sound effects (such as, for example, playing a song after the device is used correctly) and the like.

### Representative Embodiments

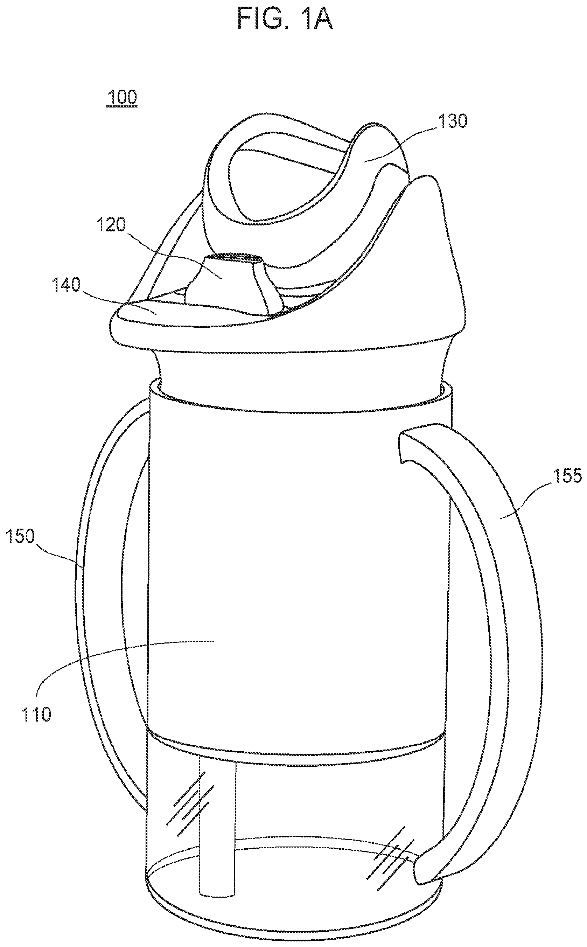

The systems having been generally reviewed, representative specific embodiments of various system configurations are now described in greater detail. As shown in FIGS. 1A to 1D, a handheld device 100 for opening eustachian tubes during drinking according to an embodiment of the disclosure includes a cup 110, a straw with a nozzle 120, a nasal mask 130 to engage the nasal cavity for pressurization, aligner elements to align the subject's face, including a chin blocker 140 to stop the child from pivoting their chin backwards, a short nozzle 120 to bring the face close to the device, nose blocking elements to ensure that the subject's nose sits inside the mask, a pressure sensor, an air pump, an accelerometer and a control system. The illustrated device 100 includes side handles 150 and 155 to provide for ease of use. The device is configured to function to automatically open the eustachian tubes during drinking. During use, the device 100 is turned on into a standby mode, the device enters a sensing mode with a low flow of air coming through the mask when it is picked up. When the subject drinks, the device senses palate closure during a swallow using nasal cavity back-pressure and performs a brief, high flow puff of air to pressurize the nasal cavity to open the eustachian tubes and ventilate the mid ear. This puff is anticipated to last for less than a second but may last for any duration. The device then registers that a treatment (nasal pressurization to open the eustachian tubes and ventilate the mid ear) has been successfully performed (and the mask is properly positioned) and goes into standby mode waiting the required time between treatments, which may vary, ranging in some instances from 0.05 s to one day. At this point the device reactivates and performs another pressurization therapy when the subject swallows again. A parent can monitor the therapy in an app, e.g., as described in greater detail below. In some embodiments, the device functions to deliver drugs to the eustachian tubes. The device may be used for Otology but can additionally or alternatively be used for any suitable applications, clinical or otherwise. The system/device can be configured and/or adapted to function for any suitable clinical intervention that requires the nasal cavity pressurization or eustachian tube opening.

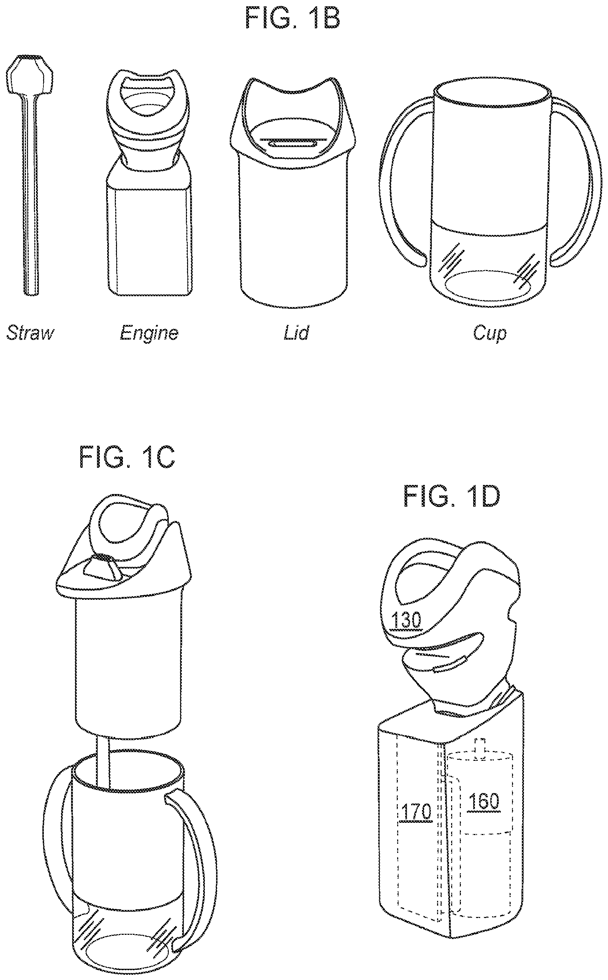

As shown in FIG. 1B, the system 100 illustrated in FIG. 1A is made up of four distinct parts, which includes a straw, an engine (which houses the controller and pressure source) and nasal mask, a lid and a cup. FIG. 1D provides a cutaway view of the engine part, which shows the nasal mask 130 (i.e., nose-piece), as well as a pressurized gas cannister 160 and a controller 170. FIG. 1C illustrates how the four disparate parts fit together to produce device 100.

As shown in FIGS. 1A to 1D, the device 100 includes a face-aligner. The face-aligner functions to align the subject's face and ensure that the subject drinks from the bottle the nose-piece is aligned appropriately to seal the nasal cavity for pressurization of the nasal cavity. The face-aligner may include a chin blocker to stop the subject from pivoting their chin backwards, a short nozzle to bring the face close to the device, nose blocking elements to ensure that the patients nose sits inside the mask and a straw that ensures that if the device is used upside down (with the chin in the nose-piece) no fluid can be extracted. Other elements that may be present include: a valve on the straw to only allow fluid to come out when the device is oriented appropriately based on feedback from an accelerometer, a bite valve that can only be used if oriented correctly in the mouth, an extra-wide nozzle that can only fit within the mouth in the correct plane, extra wide arm handles to make non-parallel positioning of the drink bottle uncomfortable and other features to maximize the ergonomics of using the device in the appropriate orientation such as angulation of the handles.

As shown in FIGS. 1A to 1D, the device includes a pressurization element. The pressurization element functions to create positive pressure in a sealed nasal cavity during a swallow. The pressurization element may be an air-pump and includes a power source, such as a battery. Alternatively, the pressurization element may be a pressure vessel with a release valve (like, but not limited to, a CO₂ cannister for bike tire inflation) or a balloon, e.g., as shown in FIG. 1D as 160.

As shown in FIGS. 1A to 1D, the device 100 includes a control system 170. The control system 170 functions to sense when the patient has picked up the device, sense when the palate is closed and the nose piece is sealed on the face and then turns on a high flow of air, until a threshold pressure is reached which opens the eustachian tube. At this point the system holds this pressure for a set duration, e.g., 0.05 s to 2 s, and then stops flow completely for a set duration, e.g., 0.05 s to 1 day, before resetting and starting again. The control system may include an accelerometer, a pressure sensor to monitor pressure at the air output, a microcontroller, a module to communicate information with a phone or similar remote device. The relevant parameters, such as threshold of opening pressure, high flow duration, back pressure threshold to indicate palate closure, may be configured to be set either by the manufacturer, the clinician, the caregiver or the subject.

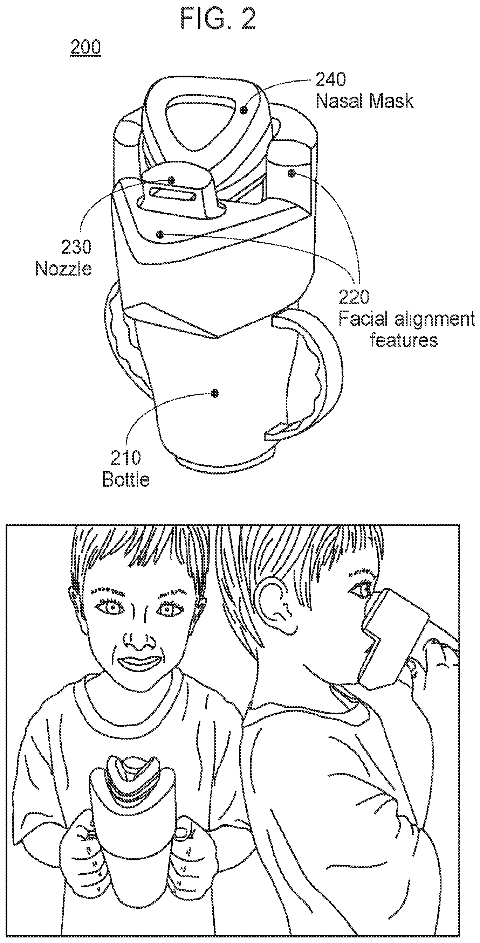

FIG. 2 provides a view of an alternative device 200, and its use. As shown in FIG. 2, device 200 includes bottle 210, facial alignment features 220, nozzle 230 and nasal mask 240. Also shown in FIG. 2 is a child holding using the device.

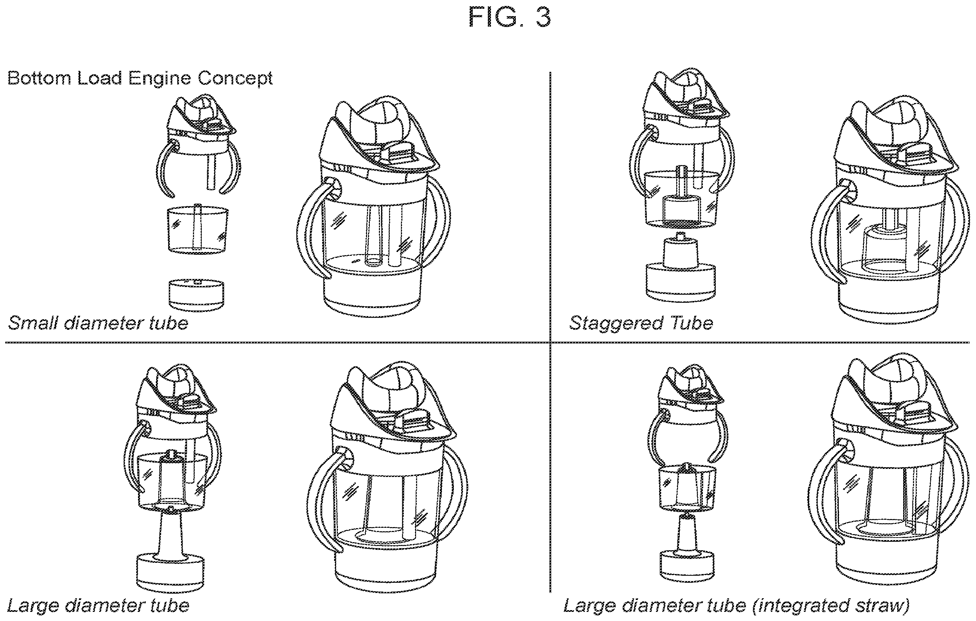

FIG. 3 provides alternative part configurations of a device. In particular, FIG. 3 illustrates alternative configurations of the electrical components of the device such that the electrical components, including a pressure source, which may comprise a motor or alternative mechanisms for applying pressure to the nasal passage, may be mounted in the base of the device and the air-path from the motor (i.e., the path through which pressurized gas from the pressure source is in fluidic communication with the subject) may travel upwards through a liquid container portion of the device. As seen in the configuration set forth in the upper left hand quadrant of FIG. 3, a small diameter tube may provide a path for compressed gas only through the liquid container (i.e., bottle) portion of the device. Alternatively, as seen in the configuration set forth in the upper right hand quadrant of FIG. 3, a staggered tube, i.e., a tube configured to partially fit the motor within the bottle may provide a path for compressed gas through the bottle, but where the motor does not extend along the entire vertical height of the bottle. As seen in the configurations set forth in the lower half of FIG. 3, a large diameter tube may provide a space to fit the motor within the tube that extends the entire vertical height of the bottle. As seen in the two potential configurations of the device in the lower half of FIG. 3, the large diameter tube may or, alternatively, may not include an integrated straw.

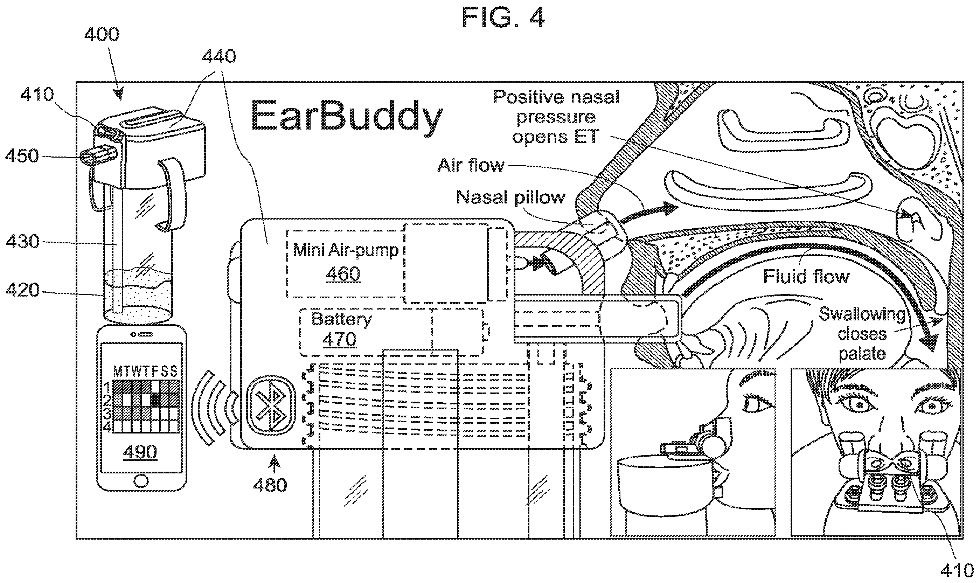

FIG. 4 illustrates an alternative embodiment of a device in accordance with an embodiment of the disclosure. In FIG. 4, device 400 includes an intra-nostril nasal passage seal 410 in the form of a nasal pillow. Also shown is bottle 420, straw 430 that exits lid 440 in a nozzle 450. The lid includes the pressure source 460 (i.e., mini Air-pump) and battery, 470, as well as a controller. The air pump is configured to force air through nasal pillow 410 into the sealed nasal passage. The lid 440 further includes a wireless (Bluetooth) communication module 480 that communicates with remote device 490 (smart phone) to display therapeutic data.

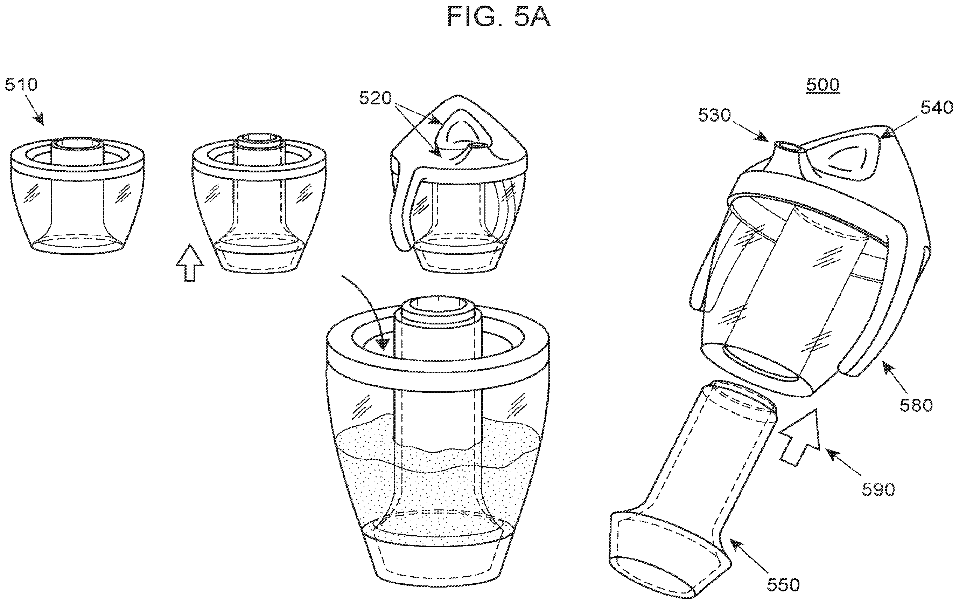

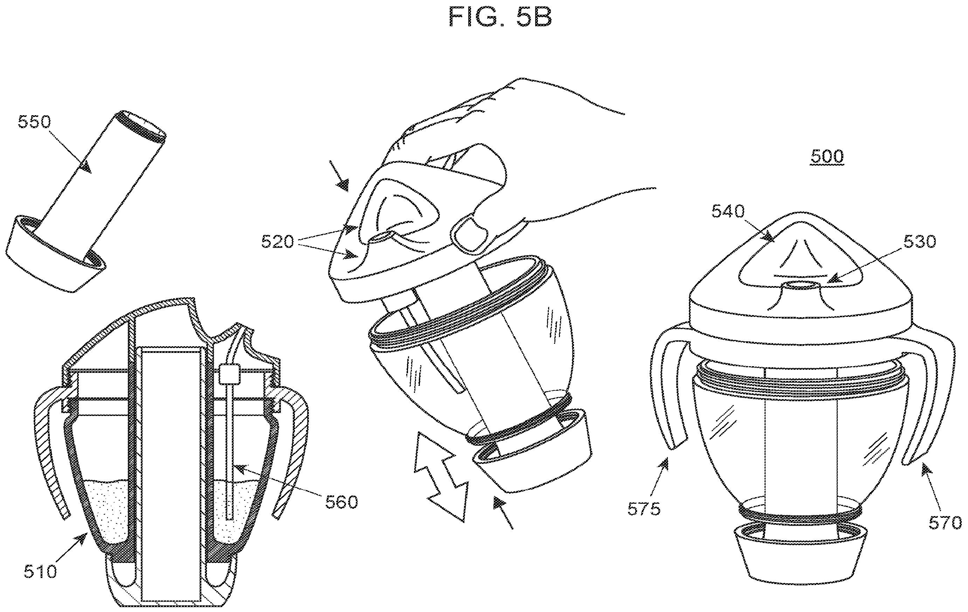

FIGS. 5A and 5B provide views of an alternative embodiment of a device 500 according to the present disclosure. As shown in FIG. 5A, device 500 includes bottle 510, facial alignment features 520, nozzle 530 and nasal mask 540. FIG. 5A illustrates a configuration of the electrical components 550 of the device 500 such that the electrical components 550, including a pressure source, which may comprise a motor or alternative mechanisms for applying pressure to the nasal passage, are separable from the bottle 510 and mounted in the base of the device 500 and the air-path from the motor may travel upwards through the bottle 510 portion of the device 500.

As seen in FIG. 5A, the system is made up of two disparate units: a first unit comprising the electrical components 550 and a second unit 580 comprising all the other elements of the system. Also seen in FIG. 5A, the second unit 580 when assembled (but without including the first unit, the electrical components 550) forms a functional sippy cup. In addition, FIG. 5A shows how the first unit can be combined with the second unit so as to turn on the electronics of the first unit. That is, assembly step 590 of combining the first unit 550 with the second unit 580 turns on the electrical components 550 of the device 500. By turns on, it is meant that the electrical components 550 are changed from an inactive state to an active or a standby state such that, for example, pressure can be applied to the nasal passage from the pressure source when the system is in use.

As shown in FIG. 5B, device 500 includes bottle 510, facial alignment features 520, nozzle 530, nasal mask 540, electrical components 550, straw 560, allowing liquid in the cup to be in fluidic communication with the nozzle, and side handles 570 and 575. FIG. 5B also illustrates a configuration of the electrical components 550 of the device 500 such that the electrical components 550, including a pressure source, such as a pressurization element, which may comprise a motor or alternative mechanisms for applying pressure to the nasal passage, may be separable from the bottle 510 and mounted in the base of the device 500 and the air-path from the motor may travel upwards through the bottle 510 portion of the device 500.

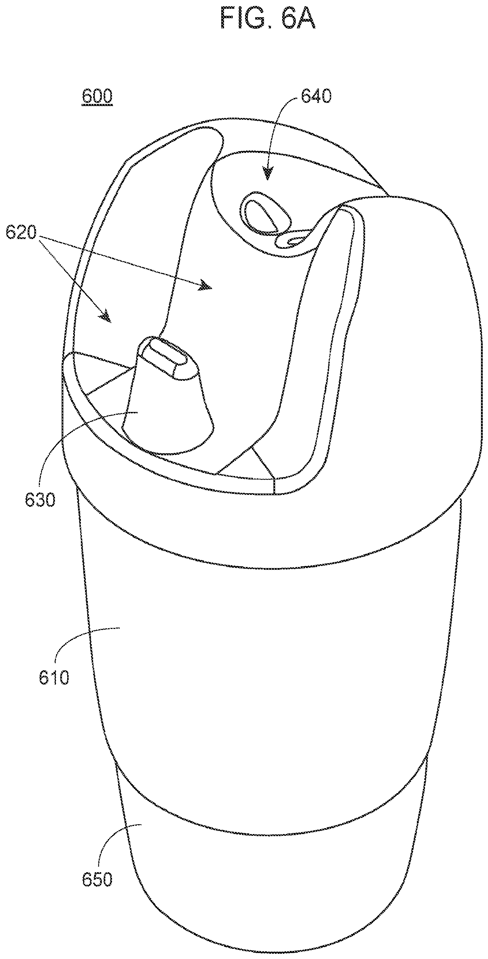

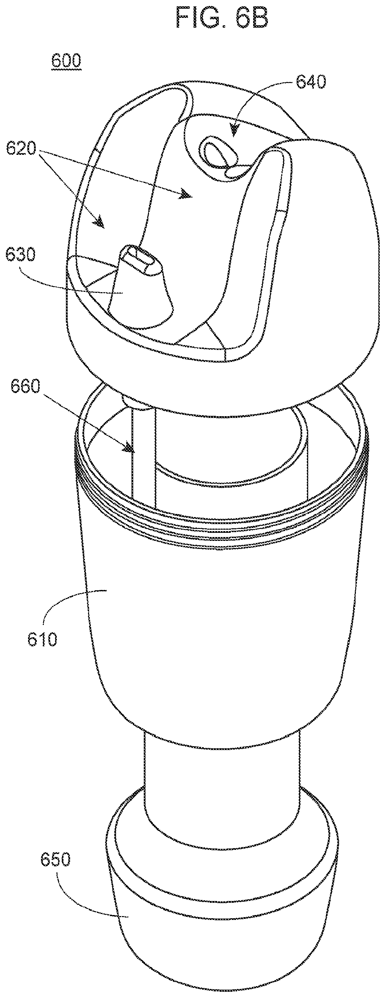

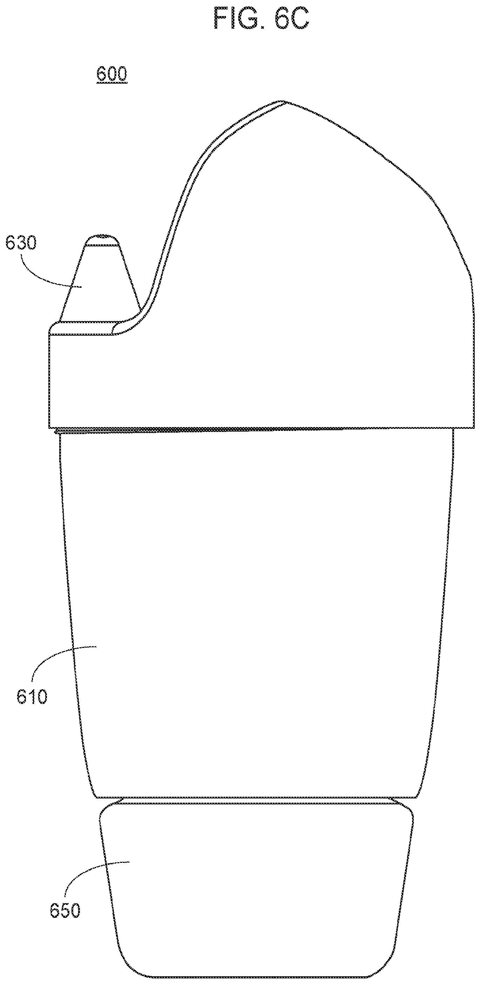

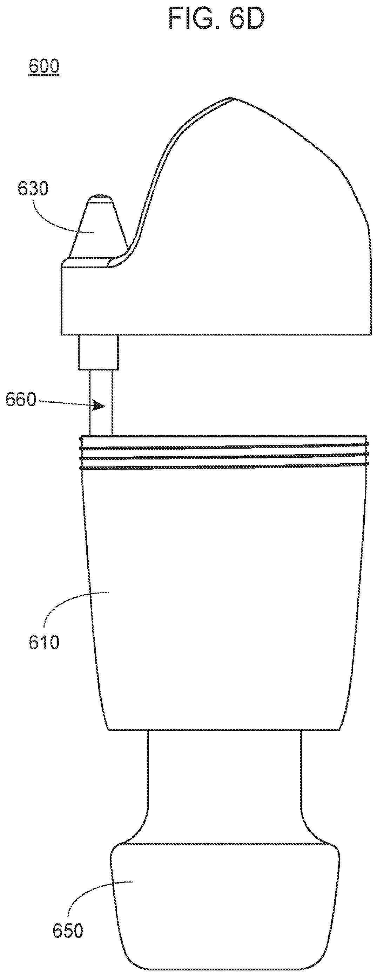

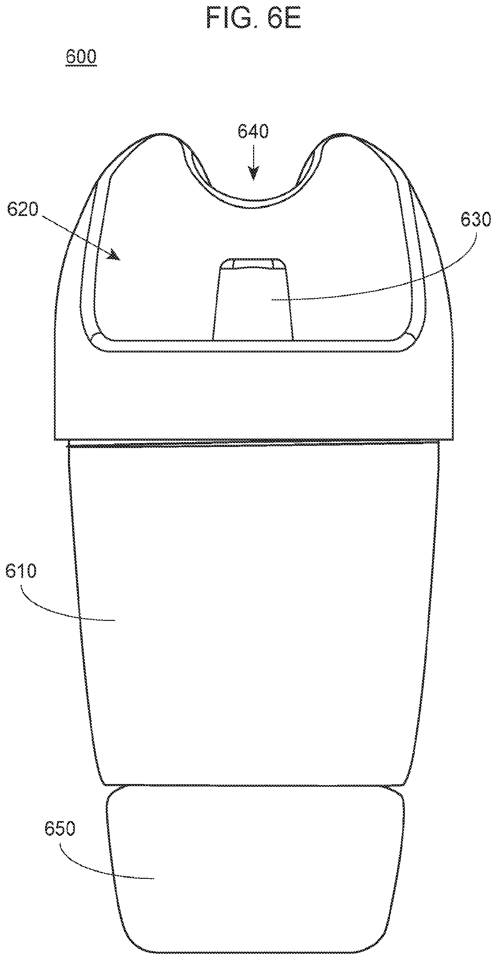

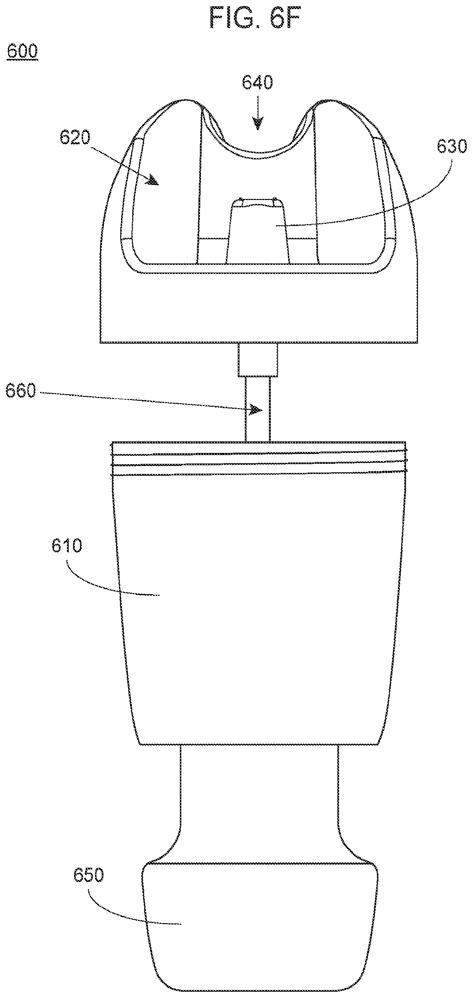

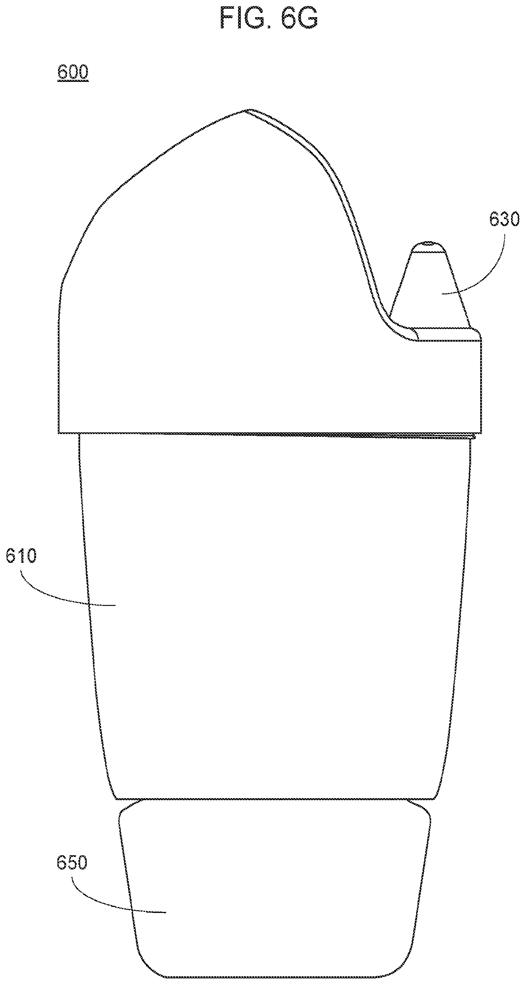

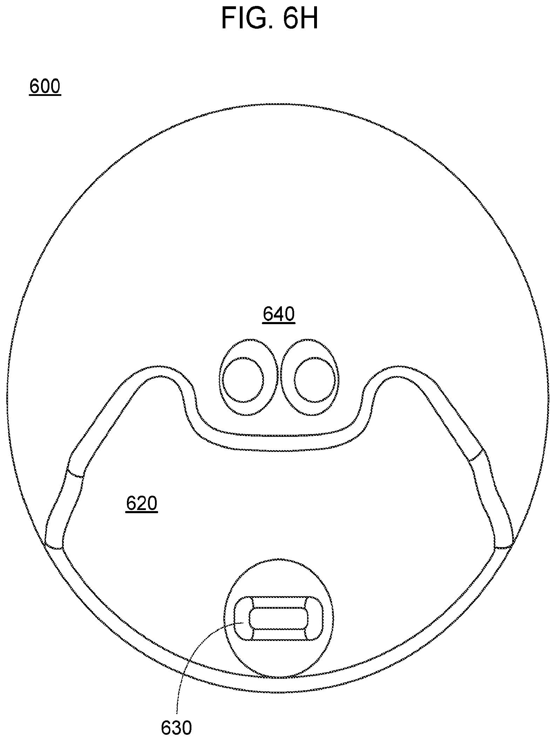

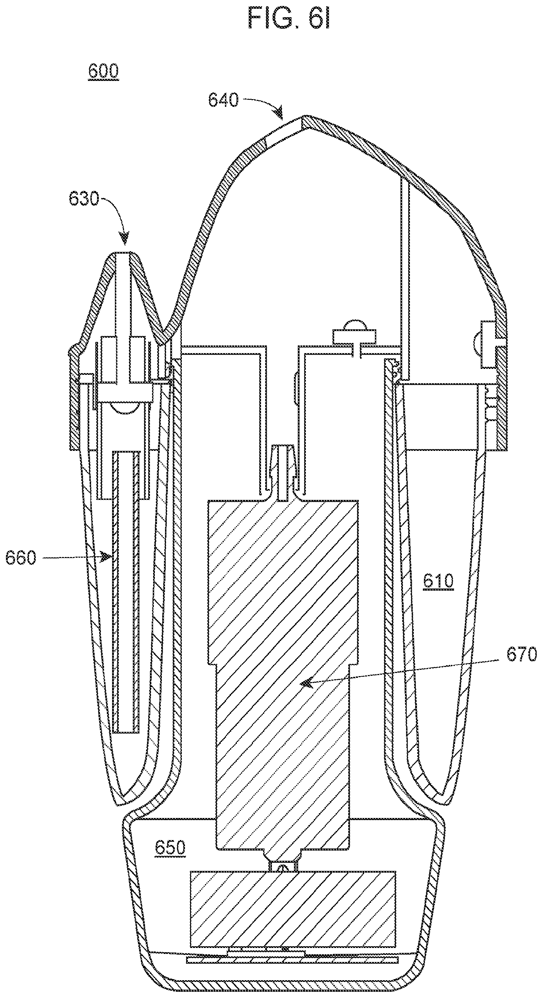

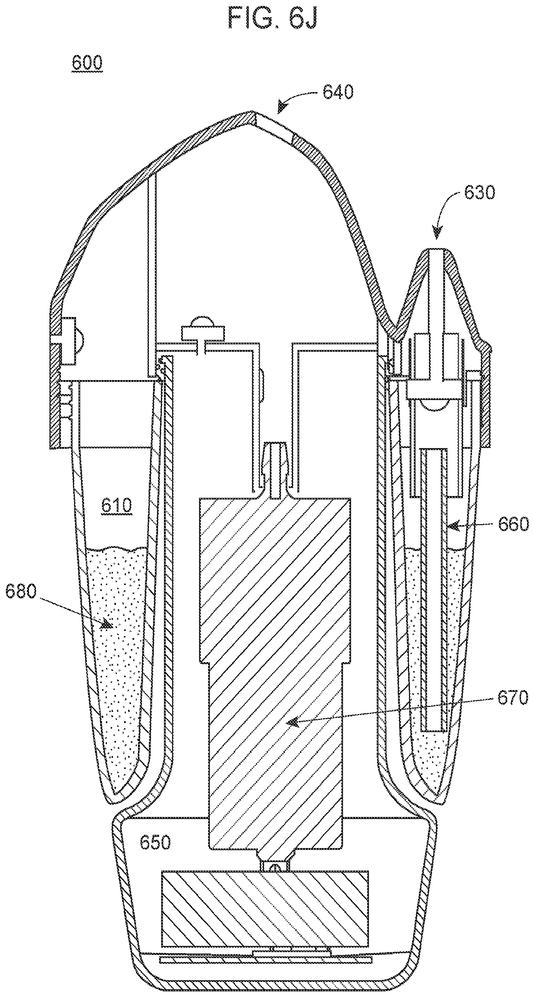

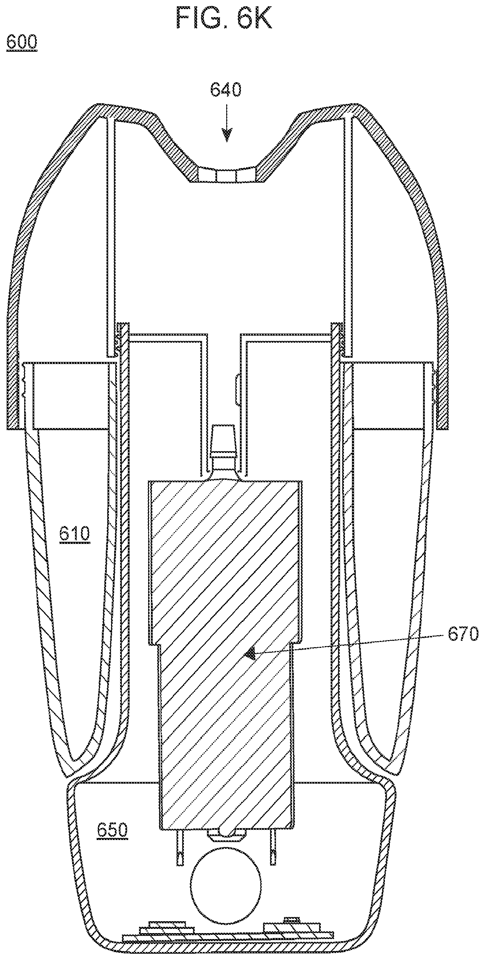

FIGS. 6A to 6K provide views of a device 600 for opening eustachian tubes during drinking according to an embodiment of the disclosure. FIG. 6A provides a perspective view of device 600. FIG. 6B provides an exploded perspective view of device 600. FIG. 6C provides a side view of device 600. FIG. 6C provides an exploded side view of device 600. FIG. 6E provides a front view of device 600. FIG. 6F provides an exploded front view of device 600. FIG. 6G provides an alternate side view of device 600. FIG. 6H provides a top view of device 600. FIG. 6I provides a cutaway side view of device 600. FIG. 6J provides an alternate cutaway side view of device 600. FIG. 6K provides a cutaway front view of device 600.

As seen in FIGS. 6A to 6K device 600 includes a bottle 610, facial alignment features 620, a nozzle 630, straw 660, nasal mask 640, engine 650, pressurization element 670 and liquid 680. As seen in FIG. 6A, bottle 610 is configured to substantially surround engine 650. Liquid 680 disposed in bottle 610 may be drunk by the subject via straw 660 and nozzle 630. Engine 650 comprises electrical components, including sensors (not shown), of device 600 as well as pressurization element 670, which is a source of positive pressure applied to the nasal passage via nasal mask 640 during use. As seen, the air path from pressurization element 670 to nasal mask 640 is through the center of device 600. Pressre source, such as pressurization element 670, may be an air-pump and includes a power source, such as a battery. Also seen in FIGS. 6A to 6K, facial alignment features 620, nozzle 630 and nasal mask 640 are configured to guide the subject's face into a position such that nasal mask 640 is engaged under the subject's nose forming a seal with each of the subject's nostrils. Facial alignment features 620, nozzle 630 and nasal mask 640 all work to align the subject's nose with nasal mask 640. Also seen in FIGS. 6A to 6K, device 600, when engine 650 is not assembled into device 600, forms a standalone, functional liquid delivery device, in particular, a sippy cup.

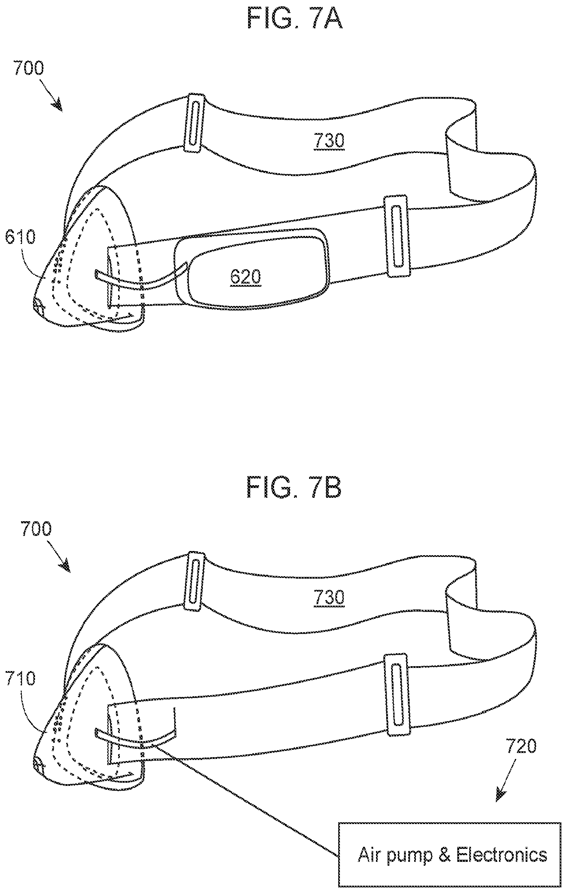

FIG. 7A illustrates an alternative embodiment of a system of the disclosure. In FIG. 7A, device 700 includes a nasal seal in the form of a nasal mask 710 that goes on the subject's face and is held in place by headstrap 730. When the subject swallows saliva, positive pressure from a pressure source 720 is introduced into the sealed nasal passage to open the eustachian tubes. Swallowing may be induced in this embodiment, where desired, by providing a separate swallow inducing element, such as a popsicle, hard candy, etc. In this embodiment, swallowing may be detected by a sensor configured to detect a parameter indicative of a swallow resultant palate closure where such a sensor may be provided separately. The sensor may detect a parameter indicative of a swallow resultant palate closure through, for example, the measurement of an increase in pressure that occurs upon swallowing. As seen in FIG. 7A, pressure source and electronics 720 are attached to headstrap 730 of device 700 such that when headstrap 730 is applied to the subject, the pressure source and electronics 720 are "worn" by the subject. The pressure source 720 may be connected to nasal mask 710 via a tube.

FIG. 7B illustrates a variation of the alternative embodiment of the disclosure shown in FIG. 7A. In FIG. 7B, device 700 also includes a nasal seal in the form of a nasal mask 710 that goes on the subject's face and is held in place by headstrap 730. When the subject swallows saliva, positive pressure from a pressure source 720 is introduced into the sealed nasal passage to open the eustachian tubes. As seen in FIG. 7B (in contrast to the embodiment depicted in FIG. 7A), pressure source and electronics 720 are not attached to headstrap 730. Instead, pressure source and electronics 720 are separate from headstrap 730. For example, in some embodiments, pressure source and electronics 720 may rest on a nearby tabletop rather than being strapped to the subject's head. Pressure source 720 may be connected to nasal mask 710 via a tube, such as a flexible tube permitting pressure source and electronics 720 to rest on, for example, a nearby tabletop, while still permitting pressure source 720 to apply pressure to the nasal mask 710.

An alternative embodiment for automatically opening eustachian tubes during drinking includes a minimum viable product embodiment that does not have any control system or pressure sensor. In such an embodiment, the system, after detecting a certain triggering event, such as, for example, that the device has been picked up, would apply a constant positive pressure from a pressure source to the nasal passage seal for introduction into the nasal passage. Such an embodiment may find use with subjects who are older children capable of closing their palate even when not using a swallow inducer.

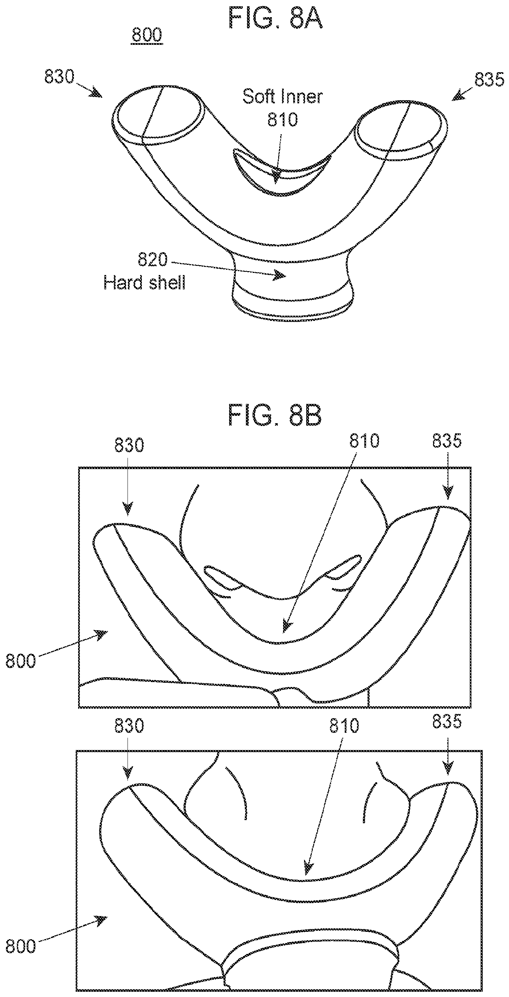

FIGS. 8A to 8D provide views of embodiments of under the nose masks 800. As seen in FIG. 8A, under the nose mask 800 is a dual durameter nose mask comprising an inner (or proximal) side 810 - a soft inner - made of relatively softer materials and an outer (or distal) side 820 - a hard shell - made of relatively harder materials. As seen in FIG. 8A, under the nose mask 800 is formed in the shape of a semicircle. The semicircle shape of under the nose mask 800 includes a first end 830 and a second end 835 that form flaps, which when the under the nose mask 800 is applied to the subject's nose, urge the center of the subject's nose to engage with the lowest point of the semicircular shaped nose mask, thereby orienting nose mask 800 into a position that results in inner side 810 of nose mask 800 sealing the nostrils.

FIG. 8B shows an embodiment of the present disclosure comprising dual durameter under the nose mask 800 in use and being engaged with a nose. The left side of FIG. 8B shows dual durameter nose mask 800 prior to being held against the nose during normal use. As seen in the image on the left side of FIG. 8B, the inner side 810 of nose mask 800 is not deformed. The right side of FIG. 8B shows dual durameter nose mask 800 while being held against the nose during normal use. As seen in FIG. 8B, when held against the nose during normal use, the inner side 810 of dual durameter under the nose mask 800 deforms to a degree that facilities conforming the shape of inner side 810 of nose mask 800 to seal each nostril. Also seen in FIG. 8B, end 830 and end 835 of nose mask 800 are positioned on the outside around either side of the nose such that the center of the nose is aligned with the center of the semicircular shape of nose mask 800.

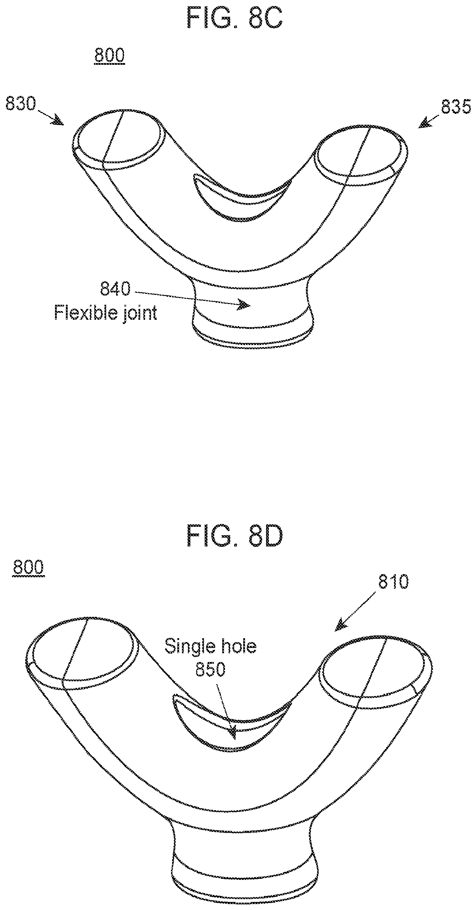

FIG. 8C shows an embodiment of the present disclosure comprising under the nose mask 800 where outer side 820 of nose mask 800 comprises flexible joint 840. Flexible joint 840 connects under the nose mask 800 with other components of the system, not shown in the figure. As described above, flexible joint 840 may be configured to allow bending of under the nose mask 800 in any direction with respect to the plane orthogonal to the axis of flexible joint 840, or, in some embodiments, substantially in an upwards and downwards direction (i.e., directions orthogonal to a line connecting end 830 with end 835). Flexible joint 840 may be used to enable under the nose mask 800 to achieve greater effectiveness across a broader population of subjects with wider variation of shapes and sizes of noses and nostrils by offering greater configurability of the orientation and positioning of under the nose mask 800 with respect to the nose and nostrils as well as the other components of the system.

FIG. 8D shows an embodiment of the present disclosure comprising under the nose mask 800 where inner side 810 of nose mask 800 comprises a single hole 850. Single hole 850 is configured to engage both nostrils such that a pressure source, not shown in the figure, of an embodiment of the system accordingly to the present disclosure, can apply positive pressure to the nasal passage sealed by the under the nose mask. In other words, as described above, single hole 850 allows a sealed nasal passage to be in fluidic communication with a pressure source, via single hold 850, such that the pressure source can apply positive pressure so as to open the eustachian tubes. Seen in FIG. 8D, under the nose mask 800 is a dual durameter under the nose mask. As such, the inner side 810 of the nose mask 800 is made of material designed to deform and thereby conform to the shape of a subject's nose nostrils. Single hole 850 is configured such that when inner side 810 of nose mask 800 is deformed, single hole 850 is shaped and positioned so as to engage both nostrils of the subject.

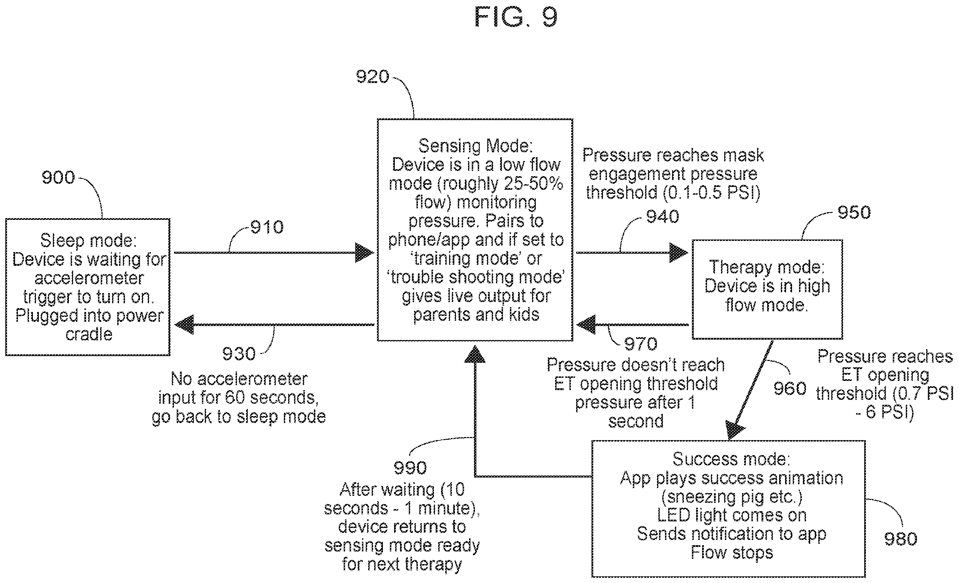

FIG. 9 depicts a control diagram for an embodiment of the system that includes an additional sensor, which may be an accelerometer, to detect when the device is in use. As seen in FIG. 9, the state labeled 900 is a "sleep mode" of the device corresponding to a period when the additional sensor detects that the device is not in use, for example when the device is not moving. For example, during sleep mode, the device may be plugged into a charging cradle. Transition labeled 910 occurs when the device transitions from "sleep mode" to a "sensing mode."

In the state referred to as "sensing mode," labeled 920, the device applies positive pressure to the nasal passage in "low flow mode," as described above, and the sensor monitors the pressure of the nasal passage. When in the "sensing mode," the device also may "pair" with a remote device such as an app running on a smart phone. Once paired with the remote device, the controller may query the remote device to determine whether the system has been set to a "training mode" or a "trouble shooting mode." In the event the additional sensor, such as an accelerometer, determines that the device has not been used for a specified amount of time, the system will transition back to "sleep mode," according to transition 930. Alternatively, in the event the sensor determines that pressure in the nasal passage reaches a specified threshold, the system proceeds according to transition 940 to the state referred to as "therapy mode."

In the state referred to as therapy mode, labeled 950, the controller causes the pressure source to increase the amount of positive pressure it is applying to the nasal passage by a specified amount (i.e., "high flow mode"). While applying increased pressure, the sensor continues to monitor the pressure of the nasal passage. In the event the monitored pressure reaches a specified pressure threshold, such as the ET opening threshold, within a specified period of time, such as within one second after increased pressure is applied by the pressure source, the system proceeds according to transition 960 to the state referred to as "success mode." On the other hand, in the event the monitored pressure fails to reach a specified pressure threshold within a specified period of time, the system proceeds according to transition 970, returning to the "sensing mode" state.

The state referred to as "success mode," labeled 980, corresponds to a successful therapy having been applied (i.e., a successful pressure event has occurred). In this state, the controller causes the pressure source to cease to apply pressure to the nasal passage. That a successful therapy was applied may be signaled to the subject in any number of ways, including, for example, the app on the remote device playing a specific animation or lights being turned on. After waiting a specified amount of time, such as, for example, ten seconds or ten minutes, the system transitions back to sensing mode, according to transition 990.

Regarding the "trouble shooting" mode referenced above, in the event that the pressure sensor, when monitoring whether nasal pressurization therapy successfully occurs, detects that therapy is not successful, then if the system is in trouble shooting mode, the remote device may provide troubleshooting feedback to the subject or caregiver based on various data sensed by the system, such as, for example, a pressure sensor, an accelerometer, and a battery level sensor. Examples of trouble shooting scenarios and the associated noncompliance state include the following:
- Sensing pressure not triggered (i.e., transition 940 fails to occur). In this case, the system may output a message to the subject or the care giver suggesting checking whether the subject is using device correctly or at all.
- Sensing pressure triggered such that the device enters therapy mode but successful pressurization not reached (i.e., transition 960 fails to occur). In this case, the system may output a message to the subject or the care giver suggesting prompting the subject to push his or her face more firmly into the nasal passage seal, or to check that the nasal passage seal is sealing, or to check that the nasal passage seal fits the subject.
- Sensing pressure reached but therapy mode disengaged due to, for example, head back drinking (i.e., transition 970 occurs). In this case, the system may output a message to the subject or the care giver suggesting instructing the subject to drink with his or her head forward.
- Sensing pressure triggered to therapy mode and successful pressurization reached but a target number of times therapy mode is triggered during each session is not met. In this case, the system may output a message to the subject or the care giver suggesting checking optimal mask sealing and fitting or check liquid levels (i.e., the swallow inducer) in the device.
- Sensing that battery voltage is low. In this case, the system may output a message to the subject or the care giver suggesting recharging the device.

The system may be configured such that if successful therapies are not detected as expected, the system may enter "trouble-shooting mode." If the system enters "trouble-shooting mode" and the instructions provided by the system to the subject or the care giver are followed but do not solve the issue, an additional layer of support may be provided. This may be in the form of human support whereby the support person is provided a video of the child using the device overlain on data from the device for the same session (such as pressure and accelerometer readings), interprets the data and communicates his or her findings to the caregiver.

When the system is in the "training mode" referenced above, the system, for example through the app running on a remote device, may guide the subject and/or care giver through directions geared toward someone using the device for the first time. In "training mode," the system may use any available sensors of the system to attempt to discern what the subject is doing with the system (e.g., how the subject is holding the system) and prompt him to complete the therapy through a specific series of steps. This mode may include certain gamification elements led by an on-screen character that responds to the child's actions (as detected by, for example, an accelerometer and/or a pressure sensor) in an engaging way and provide encouragement and praise when the subject attempts the training steps. "Training mode" may also include elements of the "trouble-shooting mode" discussed above. For example, the app may switch into "trouble-shooting mode" if the system were able to detect that it is being used incorrectly or ineffectively (such as pressure not being sensed in the nasal passage even though the subject has the system positioned on his face, or no successful therapies are being delivered). When using the system, caregivers may have the option at any time of switching from 'training mode' to 'trouble-shooting mode," for example by using buttons provided in the app running on a remote device.

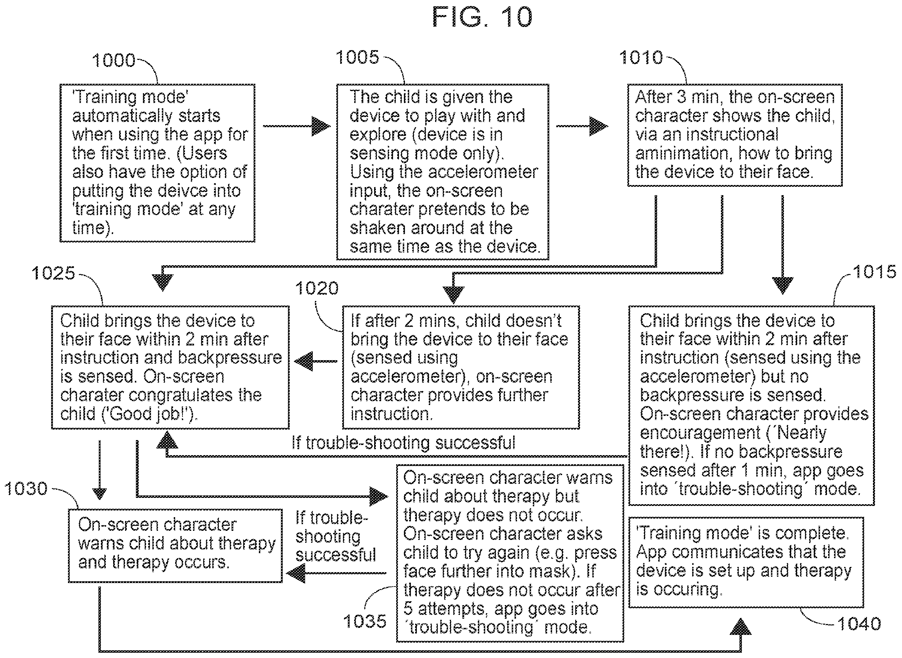

A control diagram depicting the system's states involved in "training mode" may include the those discussed below with reference to FIG. 10 for further clarity:
- Starting in the initial training mode state, labeled 1000, the system transitions to state 1005 as the subject is given an embodiment of the system to play with and explore. When in state 1005, the system would not attempt to apply any therapies to the subject. Instead, the system would collect data from available sensors only and potentially respond to sensor input via gamification aspects of an app running on a remote device. For example, if the system were shaken, an on-screen character in the app may protest and also shake about.
- From state 1005, the system transitions to state 1010 where, after a specified amount of time, for example three minutes, of the subject playing with the system, the system may begin to provide more specific instructions to the subject about how to use the device. For example, an on-screen character in the app running on the remote device may show the subject how to bring the system to his face, for example, through an instructional animation.
- From state 1010, when the system senses that the subject brings the system to his face (for example, if the sensor senses any pressure in the nasal canal), the system transitions to state 1025 wherein the system acknowledges the subject's progress, but no therapy occurs. For example, the character in the app acknowledges the correct use of the device by congratulating the subject.
- From state 1010, if the additional sensor (for example, an accelerometer) registers the expected movement of the system within a specified period of time, but no pressure is sensed by the sensor, the system transitions to state 1015 wherein the system acknowledges the subject's progress and provides additional encouragement. For example, the on-screen character may acknowledge this progress by encouraging the subject and providing further instruction.
- From state 1010, if the additional sensor (for example, an accelerometer) does not register the correct movement of the system (for example, the subject bringing the system to his face) after a specified period of time, for example, two minutes, the system transitions to state 1020 where it may provide further instructions regarding how to use the device. For example, the on-screen character may repeat the initial instructions regarding how to use the device.
- From state 1015, upon successful application of the "trouble-shooting mode," the system transitions to state 1025.
- From state 1020, after the on-screen character provides additional instruction, the system transitions to state 1025.
- From state 1025, the on-screen character provides advance notice to the subject that the therapy will commence, after which the therapy occurs. If therapy was successful, the system is in state 1030 and may provide acknowledgment and encouragement regarding the successful therapy. For example, the app may display a funny animation of the on-screen character (such as the character sneezing). If on the other hand, the therapy is not successful, the system is in state 1035 wherein the subject is encouraged to try therapy again potentially with additional instruction about how to use the system. For example, the app may display an on-screen character asking the child to try the therapy again. If the therapy is unsuccessful a specified number of times, for example if there are five unsuccessful therapies, then the system may enter trouble shooting mode, after which the system transitions to state 1030.
- Upon successful therapy being applied to the subject in state 1030, the system transitions to state 1040, wherein the app communicates to the subject and the caregiver that a successful therapy has occurred, and training mode is complete.

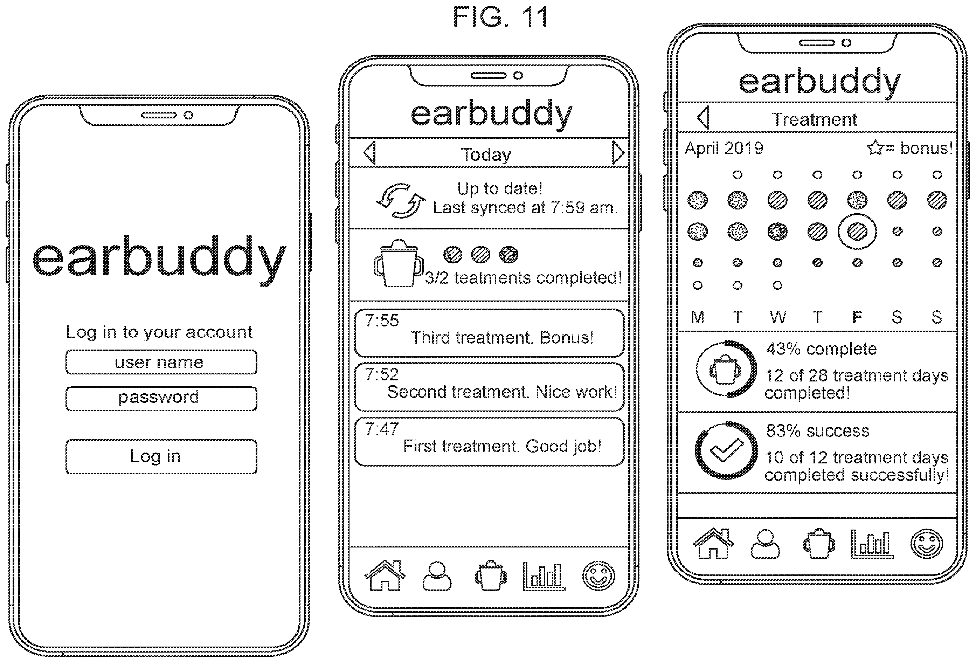

FIG. 11 provides an illustration of a graphical user interface on a smart phone that is in communication with a device of the disclosure and reports a summary of the therapy to the parents. The app may be configured to allow for push notifications. FIG. 11 illustrates examples of gamification aspects that may be employed in the remote device. For example, shown in this figure are example progress graphics indicating the day's treatments, goals and bonus treatments, in some cases indicating bonus treatments with a star. Also shown in this figure is an example calendar showing how much progress was achieved within a calendar month.

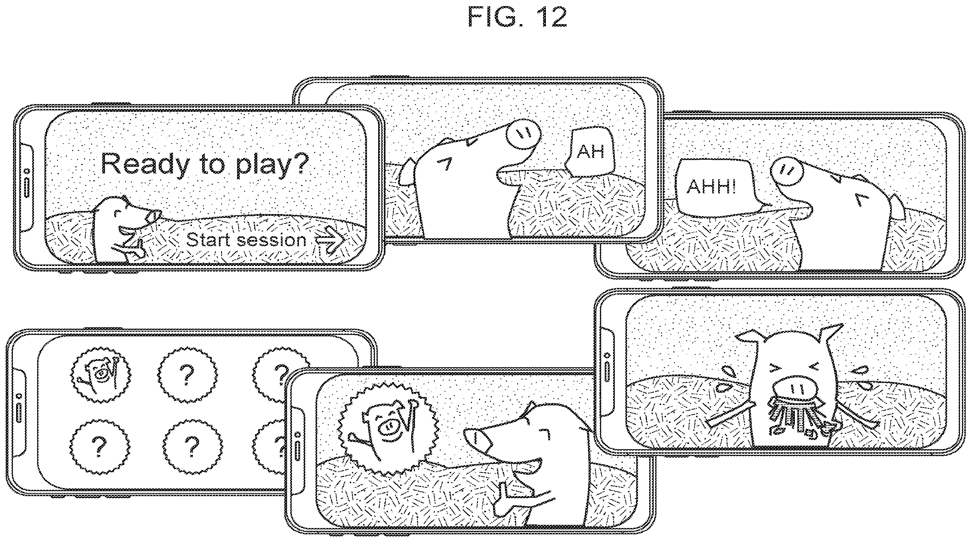

FIG. 12 provides illustrates an app that gamifies the therapy for children. This figure depicts an example of an on-screen character sneezing and providing encouragement, which, as described above, might be displayed after a therapy is successfully completed.

### Computer Implemented Embodiments

The various algorithm steps described in connection with the embodiments disclosed herein can be implemented as electronic hardware, computer software, or combinations of both. To clearly illustrate this interchangeability of hardware and software, various illustrative steps have been described above generally in terms of their functionality. Whether such functionality is implemented as hardware or software depends upon the particular application and design constraints imposed on the overall system. The described functionality can be implemented in varying ways for each particular application, but such implementation decisions should not be interpreted as causing a departure from the scope of the disclosure.

The various illustrative steps, components, and computing systems (such as devices, databases, interfaces, and engines) described in connection with the embodiments disclosed herein can be implemented or performed by a machine, such as a general purpose processor, a graphics processor unit, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general purpose processor can be a microprocessor, but in the alternative, the processor can be a controller, microcontroller, or state machine, combinations of the same, or the like. A processor can also be implemented as a combination of computing devices, e.g., a combination of a DSP and a microprocessor, a plurality of microprocessors, one or more microprocessors in conjunction with a DSP core, or any other such configuration. Although described herein primarily with respect to digital technology, a processor can also include primarily analog components. A computing environment can include any type of computer system, including, but not limited to, a computer system based on a microprocessor, a graphics processor unit, a mainframe computer, a digital signal processor, a portable computing device, a personal organizer, a device controller, and a computational engine within an appliance, to name a few.

The steps of a method, process, or algorithm, and database used in said steps, described in connection with the embodiments disclosed herein can be embodied directly in hardware, in a software module executed by a processor, or in a combination of the two. A software module, engine, and associated databases can reside in memory resources such as in RAM memory, FRAM memory, flash memory, ROM memory, EPROM memory, EEPROM memory, registers, hard disk, a removable disk, a CD-ROM, or any other form of non-transitory computer-readable storage medium, media, or physical computer storage known in the art. An exemplary storage medium can be coupled to the processor such that the processor can read information from, and write information to, the storage medium. In the alternative, the storage medium can be integral to the processor. The processor and the storage medium can reside in an ASIC. The ASIC can reside in a user terminal. In the alternative, the processor and the storage medium can reside as discrete components in a user terminal.

### METHODS

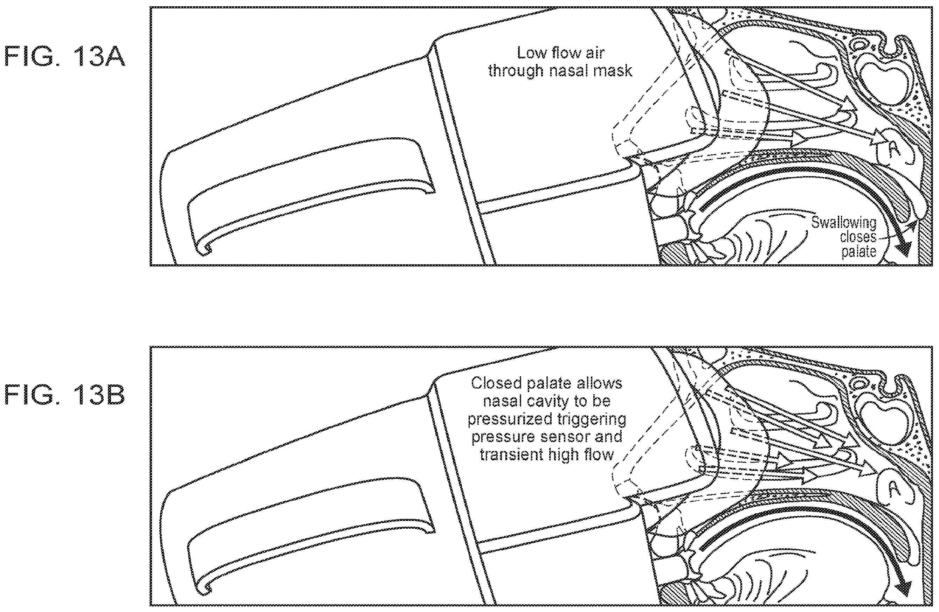

As summarized above, also provided are methods of opening eustachian tubes in a subject. In practicing methods not part of the invention, aspects of the methods include sealing the subject's nasal passage; inducing swallowing in the subject; and applying positive pressure to the sealed nasal passage upon detection of a swallow resultant palate closure. The subject's nasal passage may be sealed in a number of different ways, e.g., depending on the nature of the device employed. For example, where an extra-nostril nasal passage seal is employed, such as a nasal mask, the nasal mask may be positioned over the subject's nose so as to provide the desired air tight nasal passage seal. In other cases where an extra-nostril nasal passage seal is employed, such as an under the nose mask, the mask may be positioned on the bottom of the subject's nose so as to provide the desired air tight nasal passage seal. Alternatively, where an intra-nostril nasal passage seal is employed, the methods may include introducing a component of, e.g., nasal pillow, into the nostrils of the subject so as to provide the desired nasal passage seal. Sealing of the nasal passage using a device in accordance with the disclosure is shown in FIG. 13A.

Following sealing of the nasal passage of the subject, the subject is induced to swallow. Swallow induction may be achieved using a number of different approaches, e.g., by having the subject drink a liquid, e.g., through a straw, sip lid, nipple, etc., by having the subject suck on an ingestible composition, e.g., a popsicle, hard candy, etc., as desired.

Upon detection of a swallow resultant palate closure, e.g., by using a sensor of the system (such as a pressure sensor), such as described above, the methods include applying positive pressure to the sealed nasal passage. Positive pressure may be applied to the sealed passage using a number of different protocols, e.g., depending the particular system being employed. For example, positive pressure may be employed by introducing a volume of pressurized gas into the sealed nasal passage, e.g., through the nasal passage seal. The positive pressure may be applied to the sealed nasal passage for a predetermined application period, which may vary, ranging in some instances from 0.1 s to 1 s, such as 0.1 s to 0.2 s or 0.4 s to 0.5s or 0.9 s to 1 s. FIG. 13B illustrates positive pressure being applied to a sealed nasal passage with a closed palate to open eustachian tubes.

Where desired the methods may include introducing an active agent, such as described above, into the sealed nasal passage, and in some instances eustachian tubes insufflated by practice of the methods.

The methods and systems may be employed in any application where opening of eustachian tubes is desired. In some instances, the methods and systems are employed in the treatment of a subject, such as non-adult subject, e.g., a child, for Otitis Media with Effusion (OME). In some such instances, the subject has been diagnosed as suffering from the condition, and in some instances the subject is in the waiting period before surgery is indicated. In some instances, the methods and systems are employed in the treatment of a subject, such as a subject with eustachian tube dysfunction, an adult subject with OME, a subject with recurrent acute otitis media, subjects who are divers with ear discomfort, subjects who are flight attendants or other airline personnel with ear discomfort or a subject who is a child seeking preventive measures due to being at higher risk for OME.

### KITS

Also provided are kits that include at least one or more patches, e.g., as described above. For example, a kit may include various parts of the device, such as shown in FIG. 1B, disparate components of a system, such as a mask of FIG. 7A or 7B and swallow inducing component, such as a candy, etc. The kit components may be present in packaging, which packaging may be sterile, as desired.

Also present in the kit may be instructions for using the kit components. The instructions may be recorded on a suitable recording medium. For example, the instructions may be printed on a substrate, such as paper or plastic, etc. As such, the instructions may be present in the kits as a package insert, in the labeling of the container of the kit or components thereof (i.e. associated with the packaging or sub-packaging) etc. In other embodiments, the instructions are present as an electronic storage data file present on a suitable computer readable storage medium, e.g., portable flash drive, DVD- or CD-ROM, etc. The instructions may take any form, including complete instructions for how to use the device or as a website address with which instructions posted on the world wide web may be accessed.

Many other variations on the methods and systems described herein will be apparent from this disclosure. For example, depending on the embodiment, certain acts, events, or functions of any of the algorithms described herein can be performed in a different sequence, can be added, merged, or left out altogether (e.g., not all described acts or events are necessary for the practice of the algorithms). Moreover, in certain embodiments, acts or events can be performed concurrently, e.g., through multi-threaded processing, interrupt processing, or multiple processors or processor cores or on other parallel architectures, rather than sequentially. In addition, different tasks or processes can be performed by different machines and/or computing systems that can function together.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

Accordingly, the preceding merely illustrates the principles of the invention. It will be appreciated that those skilled in the art will be able to devise various arrangements which, although not explicitly described or shown herein, embody the principles of the invention and are included within its scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. Moreover, nothing disclosed herein is intended to be dedicated to the public regardless of whether such disclosure is explicitly recited in the claims.

The scope of the present invention, therefore, is not intended to be limited to the exemplary embodiments shown and described herein. Rather, the scope of present invention is embodied by the appended claims.

## Claims

1. A system for opening an eustachian tube of a subject, the system comprising:
a swallow inducer, wherein the swallow inducer comprises a liquid container in fluidic communication with a mouthpiece configured to be orally contacted by the subject;
a nasal passage seal (410);
a pressure source (460) configured to apply positive pressure to a sealed nasal passage; **characterized in that** the system further comprises:
a sensor configured to detect a parameter indicative of a swallow resultant palate closure; and
a controller (170) operably coupled to the pressure source (460) and the sensor, wherein the controller (170) is configured to cause the pressure source (460) to apply positive pressure to a sealed nasal passage upon detection by the sensor of the parameter indicative of a swallow resultant palate closure.

2. The system according to Claim 1, wherein the mouthpiece is configured such that fluid is drawn from a liquid container by the subject.

3. The system according to any of the preceding claims, wherein the nasal passage seal (410) comprises an extra-nostril seal.

4. The system according to Claim 3, wherein the extra-nostril seal is an under the nose mask (800).

5. The system according to Claim 4, wherein the under the nose mask (800) comprises flaps for aligning the under the nose mask (800) with the nose of the subject.

6. The system according to Claim 4 and 5, wherein the under the nose mask (800) is configured so that the shape of a proximal section deforms to seal the nasal passage.

7. The system according to any of Claims 4 to 6, wherein the under the nose mask (800) comprises a flexible element configured to allow the under the nose mask (800) to rotate.

8. The system according to any of Claims 4 to 7, wherein the under the nose mask (800) comprises a single hole configured to engage both nostrils of the subject.

9. The system according to any of the preceding claims, wherein the sensor comprises a pressure sensor.

10. The system according to any of the preceding claims, wherein the controller (170) is configured to cause the pressure source (460) to apply positive pressure to a sealed nasal passage for a predetermined application period upon detection by the sensor of the parameter indicative of a swallow resultant palate closure.

11. The system according to Claim 1, wherein the system further comprises an aligner.

12. The system according to Claim 11, wherein the aligner is configured to ensure proper alignment of the nasal passage seal (140) and the mouth of the subject.

13. The system according to any of the preceding claims, further comprising a pressure sensor operably coupled to the controller and configured to measure the pressure of the nasal passage, whereby the pressure sensor is configured to monitor whether nasal pressurization therapy successfully occurred.

## Patentansprüche

1. Ein System zum Öffnen einer Eustachischen Röhre eines Subjekts, wobei das System umfasst:
einen Schluckauslöser, wobei der Schluckauslöser einen Flüssigkeitsbehälter umfasst, der in Fluidverbindung mit einem Mundstück steht, das so konfiguriert ist, dass es vom Subjekt oral berührt werden kann;
eine Nasengangabdichtung (410);
eine Druckquelle (460), die so konfiguriert ist, dass sie Überdruck auf einen abgedichteten Nasengang ausübt;
**dadurch gekennzeichnet, dass**
das System ferner umfasst:
einen Sensor, der so konfiguriert ist, dass er einen Parameter erfasst, der auf einen durch Schlucken bedingten Gaumenverschluss hinweist; und
eine Steuereinheit (170), die funktional mit der Druckquelle (460) und dem Sensor gekoppelt ist, wobei die Steuereinheit (170) so konfiguriert ist, dass sie die Druckquelle (460) veranlasst, Überdruck auf einen abgedichteten Nasengang auszuüben, sobald der Sensor den Parameter erfasst, der auf einen durch Schlucken bedingten Gaumenverschluss hinweist.

2. Das System nach Anspruch 1, wobei das Mundstück so konfiguriert ist, dass vom Subjekt Flüssigkeit aus einem Flüssigkeitsbehälter angesaugt wird.

3. Das System nach einem der vorstehenden Ansprüche, wobei die Nasengangabdichtung (410) eine Abdichtung außerhalb der Nasenlöcher umfasst.

4. Das System nach Anspruch 3, wobei die Dichtung außerhalb der Nasenlöcher eine Unter-der-Nase-Maske (800) ist.

5. Das System nach Anspruch 4, wobei die Unter-der-Nase-Maske (800) Laschen umfasst, um die Unter-der-Nase-Maske (800) an der Nase des Subjekts auszurichten.

6. Das System nach Anspruch 4 und 5, wobei die Unter-der-Nase-Maske (800) so ausgebildet ist, dass sich die Form eines proximalen Abschnitts verformt, um den Nasengang abzudichten.

7. Das System nach einem der Ansprüche 4 bis 6, wobei die Unter-der-Nase-Maske (800) ein flexibles Element umfasst, das so ausgebildet ist, dass sich die Unter-der-Nase-Maske (800) drehen kann.

8. Das System nach einem der Ansprüche 4 bis 7, wobei die Unter-der-Nase-Maske (800) eine einzige Öffnung umfasst, die für einen Eingriff mit beiden Nasenlöchern des Subjekts konfiguriert ist.

9. Das System nach einem der vorstehenden Ansprüche, wobei der Sensor einen Drucksensor umfasst.

10. Das System nach einem der vorstehenden Ansprüche, wobei die Steuereinheit (170) so ausgelegt ist, dass sie die Druckquelle (460) veranlasst, für eine vorbestimmte Anwendungsdauer Überdruck auf einen abgedichteten Nasengang auszuüben, sobald der Sensor den Parameter erfasst, der auf einen durch Schlucken bedingten Gaumenverschluss hinweist.

11. Das System nach Anspruch 1, wobei das System ferner eine Ausrichtvorrichtung umfasst.

12. Das System nach Anspruch 11, wobei die Ausrichtvorrichtung so konfiguriert ist, dass sie eine korrekte Ausrichtung der Nasengangabdichtung (410) und des Mundes des Subjekts sicherstellt.

13. Das System nach einem der vorstehenden Ansprüche, das ferner einen Drucksensor umfasst, der funktional mit der Steuereinheit gekoppelt und so konfiguriert ist, dass er den Druck im Nasengang misst, wobei der Drucksensor so konfiguriert ist, dass er überwacht, ob die Nasendrucktherapie erfolgreich stattgefunden hat.

## Revendications

1. Système destiné à ouvrir la trompe d'Eustache d'un sujet, le système comprenant:
un inducteur de déglutition, dans lequel l'inducteur de déglutition comprend un réservoir de liquide en communication fluidique avec un embout buccal configuré pour être mis en contact oralement par le sujet;
un joint d'étanchéité pour les voies nasales (410);
une source de pression (460) configurée pour appliquer une pression positive à une voie nasale scellée;
**caractérisé en ce que**
le système comprend en outre:
un capteur configuré pour détecter un paramètre indicatif d'une fermeture du palais résultant d'une déglutition; et
un contrôleur (170) couplé de manière opérationnelle à la source de pression (460) et au capteur, dans lequel le contrôleur (170) est configuré pour amener la source de pression (460) à appliquer une pression positive à une voie nasale scellée lors de la détection par le capteur du paramètre indicatif d'une fermeture du palais résultant d'une déglutition.

2. Système selon la revendication 1, dans lequel l'embout buccal est configuré de telle sorte que le sujet aspire du fluide à partir d'un réservoir de liquide.

3. Système selon l'une quelconque des revendications précédentes, dans lequel le joint d'étanchéité pour les voies nasales (410) comprend un joint extra-narinal.

4. Système selon la revendication 3, dans lequel le joint extra-narinal est un masque sous-nasal (800).

5. Système selon la revendication 4, dans lequel le masque sous-nasal (800) comprend des rabats permettant d'aligner le masque sous-nasal (800) avec le nez du sujet.

6. Système selon les revendications 4 et 5, dans lequel le masque sous-nasal (800) est configuré de telle sorte que la forme d'une section proximale se déforme pour assurer l'étanchéité d'une voie nasale.

7. Système selon l'une quelconque des revendications 4 à 6, dans lequel le masque sous-nasal (800) comprend un élément flexible configuré pour permettre au masque sous-nasal (800) de pivoter.

8. Système selon l'une quelconque des revendications 4 à 7, dans lequel le masque sous-nasal (800) comprend un seul orifice configuré pour entrer en prise avec les deux narines du sujet.

9. Système selon l'une quelconque des revendications précédentes, dans lequel le capteur comprend un capteur de pression.

10. Système selon l'une quelconque des revendications précédentes, dans lequel le contrôleur (170) est configuré pour amener la source de pression (460) à appliquer une pression positive à une voie nasale scellée pendant une durée d'application prédéterminée dès la détection, par le capteur, du paramètre indicatif d'une fermeture du palais résultant d'une déglutition.

11. Système selon la revendication 1, dans lequel le système comprend en outre un dispositif d'alignement.

12. Système selon la revendication 11, dans lequel le dispositif d'alignement est configuré pour assurer un alignement correct entre le joint d'étanchéité pour les voies nasales (410) et la bouche du sujet.

13. Système selon l'une quelconque des revendications précédentes, comprenant en outre un capteur de pression couplé de manière opérationnelle au contrôleur et configuré pour mesurer la pression dans la voie nasale, le capteur de pression étant ainsi configuré pour surveiller si la thérapie par pressurisation nasale s'est déroulée avec succès.
